# EUROPEAN PATENT SPECIFICATION

(11) **EP 2 785 340 B1**
(45) Date of publication and mention of the grant of the patent: **15.07.2020**
(21) Application number: 12795307.3
(22) Date of filing: 27.11.2012
(51) Int. Cl.: A61K 31/4196, A61K 31/513, A61P 31/14, A61K 9/20

(54) **COMPOSITIONS AND METHODS FOR TREATING HEPATITIS C VIRUS**
VERFAHREN UND ZUSAMMENSETZUNGEN ZUR BEHANDLUNG DES HEPATITIS-C-VIRUS
COMPOSITIONS ET MÉTHODES POUR TRAITER LE VIRUS DE L'HÉPATITE C

(30) Priority: 29.11.2011 US 201161564500 P; 14.09.2012 WO PCT/US2012/055621; 28.09.2012 US 201261707459 P; 26.10.2012 US 201213661509
(43) Date of publication of application: 08.10.2014
(60) Divisional application: 20176183.0
(73) Proprietor: Gilead Pharmasset LLC, Foster City, CA 94404 (US)
(72) Inventor: CLEARY, Darryl, G., Chapel Hill, NC 27517 (US); REYNOLDS, Charles, J., Greenville, NC 27858 (US); BERREY, Miriam, Michelle, Durham, NC 27707 (US); HINDES, Robert, G., Skillman, NJ 08558 (US); SYMONDS, William, T., San Francisco, CA 94105 (US); RAY, Adrian, S., Redwood City, CA 94065 (US); MO, Hongmei, Palo Alto, CA 94303 (US); HEBNER, Christy, M., Belmont, CA 94402 (US); OLIYAI, Reza, Burlingame, CA 94010 (US); ZIA, Vahid, San Carlos, CA 94070 (US); STEFANIDIS, Dimitrios, Mountain View, CA 94040 (US); PAKDAMAN, Rowchanak, San Carlos, CA 94070 (US); CASTEEL, Melissa, Jean, Burlingame, CA 94010 (US)
(74) Representative: Wallace, Sheila Jane
(86) International application number: PCT/US2012/066605
(87) International publication number: WO 2013/082003

(56) References cited:
- WO-A1-2010/112203
- DE-A1-102008 057 284
- US-A1- 2010 267 785
- US-A1- 2010 298 257
- US-A1- 2011 251 152
- Gane, E.J. et al: "Once daily PSI-7977 plus RBV: pegylated interferon-alfa not required for complete rapid viral response in treatment-naive patients with HCV GT2 or GT3", Hepatology, vol. 54, no. 4, 34, 30 September 2011 (2011-09-30), page 377A, XP002688540, Retrieved from the Internet: URL:http://onlinelibrary.wiley.com/doi/10. 1002/hep.24666/pdf
- NELSON, D.R.: "Once daily PSI-7977 plus PEG-IFN/RBV in HCV GT1: 98% rapid virologic response, complete early virologic response:the PROTON study", JOURNAL OF HEPATOLOGY, vol. 54, 1372, March 2011 (2011-03), page s544, XP002688541,
- LALEZARI, J. ET AL.: "Once daily PSI-7977 plus PEGINF/RBV in a phase 2B trial: rapid virologic suppression in treatment-naive patients with HCV GT2/GT3", JOURNAL OF HEPATOLOGY, vol. 54, 61, March 2011 (2011-03), page s28, XP002688542,

## Description

### Field of the Invention

Disclosed herein are a composition and unit dosage form for the treatment of hepatitis C virus (HCV) infection comprising GS-7977 and at least one pharmaceutically acceptable excipient, as well as methods for making the said composition and unit dosage form. Also disclosed herein is the use of such compositions and unit dosage forms in the treatment of hepatitis C virus infection in a human. Also disclosed herein is a method of treating a subject, preferably a human, infected with hepatitis C virus, said method comprising administering to the subject for a time period an effective amount of GS-7977 and an effective amount of ribavirin. In one example, the method comprises administering to the subject an interferon-free treatment regimen comprising an effective amount of GS-7977 and an effective amount of ribavirin. In a particular example, the method is sufficient to produce an undetectable amount of HCV RNA in the subject for at least 12 weeks after the end of the time period.

### Background

Hepatitis C virus ("HCV") infection is a major health problem that leads to chronic liver disease, such as cirrhosis and hepatocellular carcinoma, in a substantial number of infected individuals, estimated by the World Health Organization to be about 3% of the world's population. (World Health Organization, Hepatitis C (2002).) According to the U.S. Centers for Disease Control and Prevention, HCV is the most common blood-borne infection in the United States, with an estimated 3.2 million people (1.8%) chronically infected in the United States alone. (U.S. Centers for Disease Control and Prevention, Viral Hepatitis Surveillance - United States, 2010; U.S. Centers for Disease Control and Prevention, Morbidity and Mortality Weekly Report 70(17): 537-539 (May 6, 2011).) An estimated 150-180 million individuals are chronically infected with HCV worldwide, with 3 to 4 million people infected each year. (World Health Organization, Hepatitis C, Fact Sheet No. 164 (July 2012); Ghany et al., Hepatology (2009) 49(4): 1335-1374.) Once infected, about 20% of people clear the virus, but the rest can harbor HCV for the rest of their lives. Ten to twenty percent of chronically infected individuals eventually develop liver-destroying cirrhosis or cancer. (Naggie et al., J. Antimicrob. Chemother. (2010) 65: 2063-2069.) The viral disease is transmitted parenterally by contaminated blood and blood products, contaminated needles, or sexually and vertically from infected mothers or carrier mothers to their offspring.

The HCV virion is an enveloped positive-strand RNA virus with a single oligoribonucleotide genomic sequence of about 9600 bases which encodes a polyprotein of about 3,010 amino acids. The protein products of the HCV gene consist of the structural proteins C, E1, and E2, and the non-structural proteins NS2, NS3, NS4A and NS4B, and NS5A and NS5B. The nonstructural ("NS") proteins are believed to provide the catalytic machinery for viral replication. The NS3 protease releases NS5B, the RNA-dependent RNA polymerase, from the polyprotein chain. HCV NS5B polymerase is required for the synthesis of a double-stranded RNA from a single-stranded viral RNA that serves as a template in the replication cycle of HCV. Therefore, NS5B polymerase is considered to be an essential component in the HCV replication complex. (K. Ishi, et al, Hepatology (1999) 29: 1227-1235; V. Lohmann, et al., Virology (1998) 249: 108-118.) Inhibition of HCV NS5B polymerase prevents formation of the double-stranded HCV RNA and therefore constitutes an attractive approach to the development of HCV-specific antiviral therapies.

A number of potential molecular targets for drug development of direct acting antivirals as anti-HCV therapeutics have now been identified including, but not limited to, the NS2-NS3 autoprotease, the N3 protease, the N3 helicase, and the NS5B polymerase. The RNA-dependent RNA polymerase is essential for replication of the single-stranded, positive sense, RNA genome, and this enzyme has elicited significant interest among medicinal chemists. Another auxiliary protein of HCV is referred to as NS5A. The NS5A nonstructural protein is a phosphoprotein, with no apparent enzymatic activity; however it acts as a multifunctional regulator of cellular pathways, including host cell growth, immunity and innate immunity, and virus replication. (Appel et al., J. Virol. (2005) 79: 3187-3194; Evans et al., Proc. Natl. Acad. Sci. USA (2004) 101: 13038-13043; Gale et al., Nature (2005) 436: 939-945; Gale et al., Virology (1997) 230: 217-227; Ghosh et al., J. Gen. Virol. (1999) 80(Pt 5): 1179-1183; Neddermann et al., J. Virol. (1999) 73: 9984-9991; Polyak et al., Hepatology (1999) 29: 1262-1271; Shimakami et al., J. Virol. (2004) 78: 2738-2748; Shirota et al., J. Biol. Chem. (2002) 277: 11149-11155; and Tan et al., Proc. Natl. Acad. Sci. U. S. A. (1999) 96: 5533-5538.) NS5A is associated with host cell membranes through its N-terminal amphipathic helix, where it is a part of the replication complex. (Elazar et al., J. Virol. (2004) 78: 11393-11400 and Penin et al., J. Biol. Chem. (2004) 279: 40835-40843.) Recent studies suggest that NS5A is organized into three domains: the first 213 amino acids in the N-terminal domain constitutes domain I and contains a zinc binding motif suggesting that the protein is a zinc metalloprotein and domains II and III are in the C-terminal region of the protein. (Tellinghuisen et al., J. Biol. Chem. (2004) 279: 48576-48587 and Tellinghuisen et al., Nature (2005) 435: 374-379.) NS5A exists in two phosphorylated forms: a basal form of 56 kD and a hyperphosphorylated form of 58 kD. The protein is phosphorylated at specific sites, primarily on serine residue within domains II and III, by host cell kinases. (Ide et al., Gene (1997) 201: 151-158; Kaneko et al., Biochem. Biophys. Res. Commun. (1994) 205: 320-326; Katze et al., Virology (2000) 278: 501-513; Reed et al., J. Biol. Chem. (1999) 274: 28011-28018; Reed et al., J. Virol. (1997) 71: 7187-7197; and Tanji et al., J. Virol. (1995) 69: 3980-3986.)

The initially-approved standard of care ("SOC") for the treatment of chronic HCV infection is a combination therapy with pegylated interferon alfa-2a or pegylated interferon alfa-2b (collectively "peginterferon" or "PEG") used alone or in combination with ribavirin ("RBV"). The primary goal of treatment for chronic hepatitis C is a sustained virologic response ("SVR"), which refers to an undetectable level of serum HCV RNA maintained for a period of time post-treatment. Host factors including age, body weight, race, and advanced fibrosis influence the outcome of treatment (Dienstag and McHutchison Gastroenterology (2006)130: 231-264 and Missiha et al., Gastroenterology (2008) 134: 1699-1714), but are poor predictors of response. In contrast, viral factors like the genotype and the on-treatment pattern of viral response can be used to determine the likelihood of treatment success and guide treatment duration individually, and they have proven to be very useful in clinical practice. (Ge et al., Nature (2009) 461: 399-401.)

In spite of an encouraging response in some patients to SOC treatment, the overall response to peginterferon/ribavirin combination therapy among patients infected with Hepatitis C virus is only about 50%. SVR rates are <50% for patients infected with genotype 1 HCV treated with a prolonged duration (48-72 weeks) of peginterferon/ribavirin therapy. (Naggie et al., J. Antimicrob. Chemother. (2010) 65: 2063-2069.) Accordingly, there is a need to provide a therapy resulting in improved SVR compared to the outcome of treatment with peginterferon alone or in combination with ribavirin. There is also a need to provide a therapy that reduces the time in which patients show evidence of complete viral suppression (negative HCV status) following the initiation of treatment.

Peginterferon alfa-2a ("PEG-IFN-α-2a" or "peginterferon α-2a"), marketed under the trademark PEGASYS®, is an antiviral administered by subcutaneous injection indicated for, among other things, treatment of chronic hepatitis C ("CHC") when administered alone or in combination with ribavirin. PEGASYS® is indicated for the treatment of CHC in patients with compensated liver disease not previously treated with interferon alpha, in patients with histological evidence of cirrhosis and compensated liver disease, and in adults with CHC/HIV co-infection. Combination therapy using PEG-IFN-α-2a and ribavirin is recommended unless the patient has contraindication to or significant intolerance to ribavirin.

Peginterferon alfa-2b ("PEG-IFN-α-2b" or "peginterferon α-2b"), marketed under the trademark PEGINTRON®, is also administered by subcutaneous injection and is indicated for use alone or in combination with ribavirin to treat CHC in patients with compensated liver disease. Like PEG-IFN-α-2a, PEG-IFN-α-2b has undesirable side effects.

Ribavirin ("RBV"), marketed under the trademark COPEGUS®, is a nucleoside analogue indicated for the treatment of CHC virus infection in combination with peginterferon in patients 5 years of age and older with compensated liver disease not previously treated with peginterferon, and in adult CHC patients co-infected with HIV. Ribavirin alone is not approved for the treatment of CHC. (COPEGUS® FDA-approved label, revised 08/2011.) Clinical trials have shown that ribavirin alone can normalize alanine aminotransferase ("ALT") levels transiently during the course of treatment in some patients with CHC infections. However, these studies have reported that ribavirin alone did not reduce HCV RNA levels during or after therapy and did not produce any sustained virologic response. (Di Bisceglie et al., Ann. Intern. Med. (1995) 123(12): 897-903; Dusheiko et al., J. Hepatology (1996) 25: 591-598; Bodenheimer, Jr., et al., Hepatology (1997) 26(2): 473-477.) One clinical study reported observing a decrease in HCV RNA from treatment with ribavirin monotherapy (1.0 to 1.2 g daily for 24 weeks); however, the observed HCV RNA decrease was transient and no patient receiving ribavirin monotherapy cleared HCV RNA. (Pawlotsky et al., Gastroenterology (2004) 126: 703-714.)

Treatment of CHC using peginterferon alone or in combination with ribavirin has several disadvantages. First and foremost, this therapy is not effective for many patients. For instance, certain Phase 3 clinical trials using the combination of peginterferon and ribavirin reported SVR rates of 54 to 63%, but additional studies show that the SVR rates may be much lower in certain populations. (Feurstadt et al., Hepatology (2010) 51(4): 1137-1143.) Second, use of peginterferon and ribavirin is associated with certain adverse events. For instance, the boxed warning on the PEGASYS® label states that use of peginterferon may cause or aggravate fatal or life-threatening neuropsychiatric, autoimmune, ischemic, and infectious disorders. (PEGASYS® (peginterferon alfa-2a) FDA-approved label, revised 09/2011.) Additionally, the boxed warning on the COPEGUS® label states that ribavirin adverse effects may include hemolytic anemia and that significant "teratogenic and embryocidal effects have been demonstrated in all animal species exposed to ribavirin." (COPEGUS® (ribavirin) FDA-approved label, revised 08/2011.) Finally, the peginterferon/ribavirin treatment protocol is quite expensive. Given these disadvantages, there has been a recognized need to develop new anti-HCV drug substances and treatment regimens.

The FDA recently approved two additional drug products for the treatment of genotype 1 CHC, boceprevir and telaprevir, both of which are HCV NS3/4 protease inhibitors. Boceprevir, marketed under the trademark VICTRELIS®, is indicated for the treatment of genotype 1 CHC infection, in combination with interferon and ribavirin, in adult patients (≥18 years of age) with compensated liver disease, including cirrhosis, who are previously untreated or who have failed previous interferon and ribavirin therapy. Telaprevir, marketed under the trademark INCIVEK®, is indicated, in combination with interferon and ribavirin, for the treatment of genotype 1 CHC in adult patients with compensated liver disease, including cirrhosis, who are treatment-naive or who have been previously treated with interferon-based treatment, including prior null responders, partial responders, and relapsers. Both boceprevir and telaprevir are approved for administration in combination with peginterferon and ribavirin only; neither is approved for monotherapy or for administration with ribavirin alone. (INCIVEK® (telaprevir) FDA-approved label, revised 06/2012; VICTRELIS® (boceprevir) FDA-approved label, revised 07/2012.)

The introduction of both boceprevir and telaprevir has increased the therapeutic options available to HCV-infected patients; however, both treatment regimens have certain disadvantages. A principle disadvantage is that the boceprevir and telaprevir regimens still require the use of peginterferon. Additional disadvantages are summarized below.

Boceprevir (used in combination with peginterferon α-2a and ribavirin) has a complicated dosing regimen, e.g., 800 mg (4 x 200 mg) three times daily (every 7 to 9 hours) with food. Moreover, late-stage clinical studies show that boceprevir used in combination with peginterferon and ribavirin results in a 66% SVR rate. (Manns et al., Liver Int'l (2012) 27-31.) Additionally, the boceprevir regimen must be administered for 48 weeks, which means that the treatment costs are quite expensive. Finally, use of boceprevir in combination with peginterferon and ribavirin is presently limited to those subjects infected with HCV genotype 1.

The telaprevir regimen (used in combination with peginterferon and ribavirin) requires a dosing regimen of 750 mg (2 x 375 mg) three times daily (7-9 hours apart) with food. An SVR rate of 79% was reported for patients receiving telaprevir in combination with peginterferon and ribavirin for 12 weeks. (Jacobson et al., New Engl. J. Med. (2011) 364: 2405-2416.) However, reports reveal that about half of the treated patients developed a skin rash or itching, and a small number of patients developed the severe Stevens-Johnson Syndrome, a life-threatening skin condition, in which case the regimen must be terminated. Finally, use of telaprevir in combination with peginterferon and ribavirin is presently limited to those subjects infected with HCV genotype 1. Although the treatment period is reduced for telaprevir as compared to that for boceprevir, the treatment costs for the two regimens are about the same.

Despite the additional options offered by the boceprevir and telaprevir regimens, these alternative treatments still have disadvantages. Further, genotype 1 patients who fail therapy with boceprevir and/or telaprevir in combination with peginterferon and ribavirin may develop undesirable NS3 protease inhibitor resistance. (E.g., Pawlotsky, Hepatology (2011) 53(5): 1742-1751.) There is a need for improved treatment regimens that are more effective, safe, tolerable, shorter in duration, and which are associated with reduced rates of viral breakthrough and/or viral resistance. In particular, there is a need for interferon-free treatment regimens that are effective for treating CHC but result in reduced side-effects compared to treatment regimens involving interferon or peginterferon. There is also a need for interferon-free treatment regimens for patients suffering from CHC infection who are interferon-ineligible or interferon-intolerant.

GS-7977 (also called sofosbuvir and formerly called PSI-7977) is a nucleotide analog prodrug currently in Phase 2/Phase 3 trials for treatment of chronic HCV infection.

Several Phase 2 clinical trials have been conducted to evaluate the efficacy, safety and tolerability of GS-7977 400 mg administered for 8 or 12 weeks with or without ribavirin and optionally peginterferon in subjects with GT1, GT2 or GT3 HCV. The results of these trials, along with the results if *in vitro* studies, revealed several potential and hereto unknown advantages of HCV treatment regimens utilizing GS-7977 in combination with ribavirin. These results provide a basis for the disclosed and claimed method and composition for treating HCV infection.

### Summary

Aspects and embodiments of the present invention are defined in the claims.

Disclosed herein are a composition and unit dosage form for the treatment of hepatitis C virus (HCV) infection comprising GS-7977 and at least one pharmaceutically acceptable excipient, as well as methods for making said composition and unit dosage form. Also disclosed herein is the use of such compositions and unit dosage forms in the treatment of hepatitis C virus infection in a human.

Also disclosed herein is a method of treating a subject, preferably a human, infected with hepatitis C virus, said method comprising administering to the subject for a time period an effective amount of GS-7977 and an effective amount of ribavirin. In one example, the method comprises administering to the subject an interferon-free treatment regimen comprising an effective amount of GS-7977 and an effective amount of ribavirin. In a particular example, the method is sufficient to produce an undetectable amount of HCV RNA in the subject for at least 12 weeks after the end of the time period.

### Brief Description of the Drawings

- **Figure 1.**: Plot of Mean HCV RNA (log₁₀ IU/mL) versus time during treatment and for up to 12 weeks after the end of treatment ("EOT") for HCV GT2/GT3 treatment-naive patients receiving a combination of GS-7977 (400 mg QD) and RBV (1000/1200 mg BID based on weight) for 12 weeks (ELECTRON Group 1).
- **Figure 2.**: Fold-change in EC₅₀ for HCV replicons containing 1b, 1a, 2a, 2b, 3a, 4a, and 5a NS5B harboring the S282T mutation (compared to the corresponding wild-type) treated with GS-7977 or ribavirin.
- **Figure 3.**: Percentage of wild-type at S282 position in HCV replicons before and after treatment with GS-7977, ribavirin, and a combination of GS-7977 and ribavirin in long-term passaging study (15-30 days).

### Detailed Description

### Definitions

The phrase "a" or "an" entity as used herein refers to one or more of that entity; for example, a compound refers to one or more compounds or at least one compound. As such, the terms "a" (or "an"), "one or more", and "at least one" can be used interchangeably herein.

The term "about" (also represented by "∼") has its plain and ordinary meaning of "approximately" except as related to an amount of GS-7977, an amount of ribavirin, or an amount of HCV RNA. As related to an amount of GS-7977, an amount of ribavirin, or an amount of HCV RNA, the qualifier "about" reflects the standard experimental error.

The terms "optional" or "optionally" as used herein means that a subsequently described event or circumstance may but need not occur, and that the description includes instances where the event or circumstance occurs and instances in which it does not.

The term "subject" as used herein means a mammal. Preferably the subject is a human.

The term "effective amount" as used herein means an amount sufficient to reduce symptoms of the HCV infection in a subject.

The term "undetectable amount" refers to an amount of HCV RNA, as determined by the assay methodology described herein, that is less than the limit of detection ("LOD") of about 15 IU/mL.

A sustained virologic response (SVR) for a patient treated according to one of the treatment regimens described herein is defined as a patient who completes the HCV treatment regimen and who has an undetectable amount of HCV RNA (i.e., < about 15 IU/mL) for a period of time post-treatment as measured in accordance with the assay methodology described herein. SVR-N is the abbreviation for sustained virologic response N weeks after completion of one of the HCV treatment regimens disclosed herein. For example, SVR-4 is the abbreviation for sustained virologic response 4 weeks after completion of one of the HCV treatment regimens disclosed herein.

The term "preparation" or "dosage form" is intended to include both solid and liquid formulations of the active compound and one skilled in the art will appreciate that an active ingredient can exist in different preparations depending on the desired dose and pharmacokinetic parameters.

The term "unit dosage form" refers to a physically discrete unit containing a predetermined quantity of the active compound. Preferred unit dosage forms are those containing a daily dose or unit daily sub-dose, or an appropriate fraction thereof, of GS-7977.

The terms "pharmaceutically acceptable excipient" and "pharmaceutical excipient" as used herein refer to a compound that is used to prepare a pharmaceutical composition, and is generally safe, non-toxic and neither biologically nor otherwise undesirable, and includes excipients that are acceptable for veterinary use as well as human pharmaceutical use.

RVR is the abbreviation for rapid virologic response and refers to an undetectable level of HCV RNA in the blood at week 4 of treatment. The occurrence of RVR has been reported to be predictive of ultimate SVR for a full treatment course of 48 weeks with peginterferon/ribavirin combination treatment in HCV GT-1 patients. (Poordad et al., Clin. Infect. Dis. (2008) 46: 78-84.)

QD means that the dose is administered once a day.

BID means that the dose is administered twice a day.

TID means that the dose is administered three times a day.

QID means that the dose is administered four times a day.

The highest activities of alanine aminotransferase (ALT) are found in hepatocytes and striated (skeletal and cardiac) muscle cells. Increased serum ALT activity can accompany hepatocellular injury or necrosis of striated muscle. With cell injury or death, ALT escapes from the cytosol. In addition, release of ALT from the cytosol can occur secondary to cellular necrosis or as a result of cellular injury with membrane damage. Determination of ALT activity is a relatively sensitive indicator of hepatic damage. Mechanisms of increased activity of ALT in serum include enzyme release from damaged cells or induction of enzyme activity, such as increased enzyme synthesis from drug administration. (Zeuzem, et al., Aliment Pharmacol Ther. 2006 Oct 15; 24(8) 1133-1149).

The interleukin 28B (IL28B) gene encodes a cytokine distantly related to type I interferons and the IL-10 family. The IL28B gene, interleukin 28A (IL28A), and interleukin 29 (IL29) are three closely related cytokine genes that form a cytokine gene cluster on a chromosomal region mapped to 19q13. Expression of the cytokines encoded by the three genes can be induced by viral infection. All three cytokines have been shown to interact with a heterodimeric class II cytokine receptor that consists of interleukin 10 receptor, beta (IL10RB), and interleukin 28 receptor, alpha (IL28RA). (National Center for Biotechnology Information, Entrez Gene Entry for IL28B, Gene ID: 282617, updated on 23-Oct-2010.)

Body mass index ("BMI") is a measurement based on a person's weight and height and is used to estimate a healthy body weight based on a person's height, assuming an average body composition. The units of BMI are kg/m².

LOD is the abbreviation for limit of detection. As used herein with regard to HCV RNA measurements, in one aspect LOD is from about 1 IU/mL to about 60 IU/mL, more preferably from about 5 IU/mL to about 30 IU/mL, and even more preferably from about 10 IU/mL to about 20 IU/mL. In a particularly preferred embodiment, the LOD is about 15 IU/mL.

GT is the abbreviation for genotype.

IU is the abbreviation for international unit, which is a measure of the amount of a substance based on biological activity or effect.

There are several recognized HCV Genotypes (1, 2, 3, 4, 5, 6, 7, 8, 9, 10 and 11), which can be further categorized by different sub-types: 1 (1a, 1b, and 1c), 2 (2a, 2b, 2c), 3 (3a and 3b), 4 (4a, 4b, 4c, 4d, and 4e), 5 (5a), 6 (6a), 7 (7a and 7b), 8 (8a and 8b), 9 (9a), 10 (10a), and 11 (11a). Genotype 1 is the predominant form found in North and South America, Europe, Asia, Australia, and New Zealand. Genotypes 2 and 3 are also widely distributed throughout North America, Europe, Australia, East Asia and some portions of Africa. In some portions of Africa, Genotype 4 predominates, while in others (such as South Africa) genotype 5 predominates. The method disclosed herein is contemplated to be independently effective for the treatment of each of the HCV genotypes, and in particular each genotype-sub-type.

The term "interferon-free" as used herein refers to a treatment regimen that does not involve the administration of interferon or pegylated interferon to the subject.

GS-7977, (S)-isopropyl 2-(((S)-(((2R,3R,4R,5R)-5-(2,4-dioxo-3,4-dihydropyrimidin-1(2H)-yl)-4-fluoro-3-hydroxy-4-methyltetrahydrofuran-2-yl)methoxy)(phenoxy)phosphoryl)amino)propanoate, available from Gilead Sciences, Inc., is described and claimed in U.S. Patent No. 7,964,580. (*See also* US 2010/0016251, US 2010/0298257, US 2011/0251152 and US 2012/0107278.) GS-7977 has the structure:

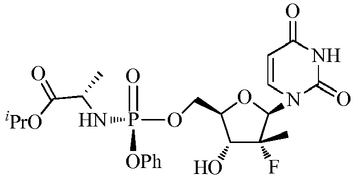

GS-7977 can be crystalline or amorphous. Examples of preparing crystalline and amorphous forms of GS-7977 are disclosed in US 2010/0298257 (US 12/783,680) and US 2011/0251152 (US 13/076,552). Polymorphic Forms 1-6 of GS-7977 disclosed in US 2010/0298257 and/or US 2011/0251152 have the following characteristic X-ray powder diffraction (XRPD) pattern 2θ-values measured according to the XRPD methods disclosed therein:
(1) 2θ-reflections (°) at about: 5.2, 7.5, 9.6, 16.7, 18.3, and 22.2 (Form 1);
(2) 2θ-reflections (°) at about: 5.0, 7.3, 9.4, and 18.1 (Form 1);
(3) 2θ-reflections (°) at about: 4.9, 6.9, 9.8, 19.8, 20.6, 24.7, and 26.1 (Form 2);
(4) 2θ-reflections (°) at about: 6.9, 9.8, 19.7, 20.6, and 24.6 (Form 3);
(5) 2θ-reflections (°) at about: 5.0, 6.8, 19.9, 20.6, 20.9, and 24.9 (Form 4);
(6) 2θ-reflections (°) at about: 5.2, 6.6, 7.1, 15.7, 19.1, and 25.0 (Form 5); and
(7) 2θ-reflections (°) at about: 6.1, 8.2, 10.4, 12.7, 17.2, 17.7, 18.0, 18.8, 19.4, 19.8, 20.1, 20.8, 21.8, and 23.3 (Form 6).

Polymorphic Forms 1 and 6 are alternatively characterized by the following characteristic XRPD pattern 2θ-values measured according to the methods disclosed in US 2010/0298257 (US 12/783,680) and US 2011/0251152 (US 13/076,552):
(1) 2θ-reflections (°) at about: 5.0 and 7.3 (Form 1); and
(2) 2θ-reflections (°) at about: 6.1 and 12.7 (Form 6).

The disclosed composition comprises polymorphic Form 6 of GS-7977. It has been found that Form 6 has a melt onset of approximately 121°C and is not hygroscopic, with less than 0.2% moisture sorption at room temperature and 90% RH. Form 6 is chemically stable when stored under opened conditions at 40°C/75% RH for 30 days.

In one aspect, GS-7977 is substantially free from its corresponding phosphorous-based diastereomer (S)-isopropyl 2-(((R)-(((2R,3R,4R,5R)-5-(2,4-dioxo-3,4-dihydropyrimidin-1(2H)-yl)-4-fluoro-3-hydroxy-4-methyltetrahydrofuran-2-yl)methoxy)(phenoxy)phosphoryl)amino)propanoate. In one embodiment, GS-7977 is at least 95% free from its corresponding phosphorous-based diastereomer. In another embodiment, GS-7977 is at least 97% free from its corresponding phosphorous-based diastereomer. In another embodiment, GS-7977 is at least 99% free from its corresponding phosphorous-based diastereomer. In a further embodiment, GS-7977 is at least 99.9% free from its corresponding phosphorous-based diastereomer.

Ribavirin, 1-β-D-ribofuranosyl-1H-1,2,4-triazole-3-carboxamide, is described in the Merck Index (12th Edition), monograph no. 8365. (*See also* U.S. Patent No. 4,530,901.)

As used herein, "treatment" or "treating" is an approach for obtaining beneficial or desired clinical results. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. "Treatment" is an intervention performed with the intention of preventing the development or altering the pathology of a disorder. The term "treatment" of an HCV infection, as used herein, also includes treatment or prophylaxis of a disease or a condition associated with or mediated by HCV infection, or the clinical symptoms thereof.

### Compositions and Unit Dosage Forms

A first embodiment is directed to a composition for the treatment of hepatitis C virus (HCV) comprising a) GS-7977, and b) a pharmaceutically acceptable excipient.

The composition for the treatment of HCV comprises from about 25% to about 35% w/w of GS-7977. In an aspect of the first embodiment, the composition comprises from about 30% to about 35% w/w of GS-7977. In another aspect, the composition comprises from about 25%, about 26%, about 27%, about 28%, about 29%, about 30%, about 31%, about 32%, about 33%, about 34%, or about 35% w/w of GS-7977. In one subembodiment, the composition comprises about 30% w/w of GS-7977. In another subembodiment, the composition comprises about 33% w/w of GS-7977. In another subembodiment, the composition comprises about 33.33% w/w of GS-7977.

The composition comprises crystalline GS-7977 having XRPD 2θ-reflections (°) at about: 6.1, 8.2, 10.4, 12.7, 17.2, 17.7, 18.0, 18.8, 19.4, 19.8, 20.1, 20.8, 21.8, and 23.3.

The pharmaceutically acceptable excipient comprises a diluent, a disintegrant, a glidant, and a lubricant.

The diluent is a combination of mannitol and microcrystalline cellulose.

The disintegrant is croscarmellose sodium (e.g., Ac-Di-Sol®).

The glidant comprises colloidal silicon dioxide.

The lubricant is magnesium stearate.

The pharmaceutically acceptable excipient comprises: a) about 30% w/w of mannitol and about 30% w/w of microcrystalline cellulose; b) about 5 % w/w of croscarmellose sodium; c) about 0.5% w/w of colloidal silicon dioxide; and d) about 1.5% w/w of magnesium stearate.

In a first aspect of the first embodiment, the composition further comprises a coating agent. In one subembodiment, the coating agent is formed from an aqueous film coat composition, wherein the aqueous film coat composition comprises a film-forming polymer, water and/or an alcohol as a vehicle, and optionally one or more adjuvants such as are known in the film-coating art. In another subembodiment, the coating agent is selected from the group consisting of hydroxypropylmethylcellulose, hydroxypropylcellulose, methylcellulose, ethylcellulose, hydroxyethylcellulose, cellulose acetate phthalate, sodium ethyl cellulose sulfate, carboxymethyl cellulose, polyvinylpyrolidone, zein, and an acrylic polymer (e.g., methacrylic acid/methacrylic acid ester copolymers such as methacrylic acid/methylmethacrylate copolymers, etc.), and a polyvinyl alcohol. In another subembodiment, the coating agent comprises a polyvinyl alcohol.

In a second aspect of the first embodiment, the composition comprises about 25% w/w to about 35% w/w of crystalline GS-7977; about 30% w/w of mannitol and about 30% w/w of microcrystalline cellulose; about 5 %w/w of croscarmellose sodium; about 0.5% w/w of colloidal silicon dioxide; and about 1.5% w/w of magnesium stearate. In one subembodiment, the composition comprises about 30% w/w to about 35% w/w of crystalline GS-7977; about 30% w/w of mannitol and about 30% w/w of microcrystalline cellulose; about 5 %w/w of croscarmellose sodium; about 0.5% w/w of colloidal silicon dioxide; and about 1.5% w/w of magnesium stearate. In another subembodiment, the composition comprises about 30% w/w of crystalline GS-7977; about 30% w/w of mannitol and about 30% w/w of microcrystalline cellulose; about 5 %w/w of croscarmellose sodium; about 0.5% w/w of colloidal silicon dioxide; and about 1.5% w/w of magnesium stearate. In one subembodiment, the composition comprises about 30% w/w of crystalline GS-7977 having XRPD 2θ-reflections (°) at about 6.1, 8.2, 10.4, 12.7, 17.2, 17.7, 18.0, 18.8, 19.4, 19.8, 20.1, 20.8, 21.8, and 23.3; about 30% w/w of mannitol and about 30% w/w of microcrystalline cellulose; about 5 %w/w of croscarmellose sodium; about 0.5% w/w of colloidal silicon dioxide; and about 1.5% w/w of magnesium stearate. In another subembodiment, the composition comprises about 33% w/w of crystalline GS-7977 having XRPD 2θ-reflections (°) at about 6.1, 8.2, 10.4, 12.7, 17.2, 17.7, 18.0, 18.8, 19.4, 19.8, 20.1, 20.8, 21.8, and 23.3; about 30% w/w of mannitol and about 30% w/w of microcrystalline cellulose; about 5 %w/w of croscarmellose sodium; about 0.5% w/w of colloidal silicon dioxide; and about 1.5% w/w of magnesium stearate. In another subembodiment, the composition further comprises a coating agent.

A second embodiment is directed to a unit dosage form for the treatment of hepatitis C virus (HCV), said composition comprising a) about 400 mg of GS-7977, and b) a pharmaceutically acceptable excipient.

The unit dosage form comprises crystalline GS-7977 having XRPD 2θ-reflections (°) at about: 6.1, 8.2, 10.4, 12.7, 17.2, 17.7, 18.0, 18.8, 19.4, 19.8, 20.1, 20.8, 21.8, and 23.3.

The pharmaceutically acceptable excipient comprises a diluent, a disintegrant, a glidant, and a lubricant.

In a one subembodiment, diluent is selected from the group consisting of calcium carbonate, dicalcium phosphate, dry starch, calcium sulfate, cellulose, compressible sugars, confectioner's sugar, dextrates, dextrin, dextrose, dibasic calcium phosphate dihydrate, glyceryl palmitostearate, hydrogenated vegetable oil (type I), inositol, kaolin, lactose, magnesium carbonate, magnesium oxide, maltodextrin, mannitol, microcrystalline cellulose, polymethacrylates, potassium chloride, powdered cellulose, powdered sugar, pregelatinized starch, sodium chloride, sorbitol, starch, sucrose, sugar spheres, talc, tribasic calcium phosphate, and combinations thereof. In a preferred subembodiment, the diluent is selected from the group consisting of dicalcium phosphate, cellulose, compressible sugars, dibasic calcium phosphate dehydrate, lactose, mannitol, microcrystalline cellulose, starch, tribasic calcium phosphate, and combinations thereof In another preferred subembodiment, the diluent is selected from the group consisting of mannitol, microcrystalline cellulose, and combinations thereof.

In another subembodiment, the disintegrant is selected from the group consisting of agar, alginic acid, bentonite, carboxymethylcellulose calcium, carboxymethylcellulose sodium, carboxymethylcellulose, cellulose, a cation exchange resin, cellulose, gums, citrus pulp, colloidal silicon dioxide, com starch, croscarmellose sodium (e.g., Ac-Di-Sol®), crospovidone, guar gum, hydrous aluminum silicate, an ion exchange resin (e.g., polyacrin potassium), magnesium aluminum silicate, methyl cellulose, microcrystalline cellulose, modified cellulose gum, modified corn starch, montmorillonite clay, natural sponge, polyacrilin potassium, potato starch, powdered cellulose, povidone, pregelatinized starch, sodium alginate, sodium bicarbonate in admixture with an acidulant such as tartaric acid or citric acid, sodium starch glycolate, starch, silicates (e.g., Veegum® HV), and combinations thereof. In a preferred subembodiment, the disintegrant is selected from the group consisting of croscarmellose sodium (e.g., Ac-Di-Sol), crospovidone, microcrystalline cellulose, modified corn starch, povidone, pregelatinized starch, sodium starch glycolate, and combinations thereof. In another preferred subembodiment, the disintegrant is croscarmellose sodium (e.g., Ac-Di-Sol).

In another subembodiment, the glidant is selected from the group consisting of colloidal silicon dioxide, talc, starch, starch derivatives, and combinations thereof. In a preferred subembodiment, the glidant comprises colloidal silicon dioxide.

In another subembodiment, the lubricant is selected from the group consisting of calcium stearate, glyceryl monostearate, glyceryl palmitostearate, hydrogenated castor oil, hydrogenated vegetable oil, light mineral oil, magnesium stearate, mineral oil, polyethylene glycol, sodium benzoate, sodium lauryl sulfate, sodium stearyl fumarate, stearic acid, talc, zinc stearate, and combinations thereof. In a preferred subembodiment, the lubricant is selected from the group consisting of calcium stearate, magnesium stearate, polyethylene glycol, sodium stearyl fumarate, stearic acid, talc, and combinations thereof. In another preferred subembodiment, the lubricant is magnesium stearate.

The pharmaceutically acceptable excipient comprises: a) about 660 mg to about 780 mg of a diluent; b) about 30 mg to about 90 mg of a disintegrant; c) about 3 mg to about 9 mg of a glidant; and d) about 15 mg to about 21 mg of a lubricant. In a preferred subembodiment, the pharmaceutically acceptable excipient comprises a) about 710 mg to about 720 mg of a diluent; b) about 60 mg of a disintegrant; c) about 6 mg of a glidant; and d) about 18 mg of a lubricant. In another preferred subembodiment, the pharmaceutically acceptable excipient comprises a) about 716 mg of a diluent; b) about 60 mg of a disintegrant; c) about 6 mg of a glidant; and d) about 18 mg of a lubricant. In another preferred subembodiment, the pharmaceutically acceptable excipient comprises a) about 710 mg to about 720 mg of a diluent comprising mannitol and/or microcrystalline cellulose; b) about 60 mg of croscarmellose sodium; c) about 6 mg of colloidal silicon dioxide; and d) about 18 mg of magnesium stearate. In another preferred subembodiment, the pharmaceutically acceptable excipient comprises a) about 716 mg of a diluent comprising mannitol and/or microcrystalline cellulose; b) about 60 mg of croscarmellose sodium; c) about 6 mg of colloidal silicon dioxide; and d) about 18 mg of magnesium stearate. In another preferred subembodiment, the pharmaceutically acceptable excipient comprises a) about 360 mg of mannitol and about 356 mg of microcrystalline cellulose; b) about 60 mg of croscarmellose sodium; c) about 6 mg of colloidal silicon dioxide; and d) about 18 mg of magnesium stearate.

In a first aspect of the second embodiment, the unit dosage form further comprises a coating agent. In one subembodiment, the coating agent further comprises a taste-masking agent. In one subembodiment, the coating agent is formed from an aqueous film coat composition, wherein the aqueous film coat composition comprises a film-forming polymer, water and/or an alcohol as a vehicle, and optionally one or more adjuvants such as are known in the film-coating art. In another subembodiment, the coating agent is selected from among hydroxypropylmethylcellulose, hydroxypropylcellulose, methylcellulose, ethylcellulose, hydroxyethylcellulose, cellulose acetate phthalate, sodium ethyl cellulose sulfate, carboxymethyl cellulose, polyvinylpyrolidone, zein, and an acrylic polymer (e.g., methacrylic acid/methacrylic acid ester copolymers such as methacrylic acid/methylmethacrylate copolymers, etc.), and a polyvinyl alcohol. In another subembodiment, the coating agent comprises a polyvinyl alcohol. In another subembodiment, the unit dosage comprises about 24 mg to about 60 mg of a coating agent. In another subembodiment, the unit dosage comprises about 36 mg to about 48 mg of a coating agent. In another subembodiment, the unit dosage comprises about 36 mg of a coating agent. In another subembodiment, the unit dosage comprises about 36 mg of a coating agent that further comprises a taste-masking agent.

In a second aspect of the second embodiment, the unit dosage form comprises about 400 mg of crystalline GS-7977 having XRPD 2θ-reflections (°) at about 6.1, 8.2, 10.4, 12.7, 17.2, 17.7, 18.0, 18.8, 19.4, 19.8, 20.1, 20.8, 21.8, and 23.3; about 360 mg of mannitol and about 356 mg of microcrystalline cellulose; about 60 mg of croscarmellose sodium; about 6 mg of colloidal silicon dioxide; and about 18 mg of magnesium stearate.

In a third aspect of the second embodiment, the unit dosage form comprises a capsule or a tablet. In one subembodiment, the unit dosage form comprises a tablet. In another subembodiment, the unit dosage form comprises a tablet and further comprises a coating agent.

With respect to the coating agent, film-forming polymers are typically provided in either aqueous or organic solvent-based solutions or aqueous dispersions. However, the polymers may be provided in dry form, alone or in a powdery mixture with other components (e.g., a plasticizer and/or colorant), which is made into a solution or dispersion by the user by admixing with the aqueous vehicle.

It will be appreciated that the aqueous film coat composition further comprises water as a vehicle for the other components, to facilitate their delivery to the surface of the unit dosage form. The vehicle may optionally further comprise one or more water soluble solvents, e.g., an alcohol and/or a ketone. Examples of an alcohol include but are not limited to methanol, isopropanol, propanol, etc. A non-limiting example for the ketone is acetone. The skilled artisan can select appropriate vehicle components to provide good interaction between the film-forming polymer and the vehicle to ensure good film properties. In general, polymer-vehicle interaction is designed to yield maximum polymer chain extension to produce films having the greatest cohesive strength and thus mechanical properties. The components are also selected to provide good deposition of the film-forming polymer onto the surface of the unit dosage form, such that a coherent and adherent film is achieved.

Suitable aqueous film coating compositions include those commercially available from Colorcon, Inc. of West Point, Pa., under the trade name OPADRY and OPADRY II (non-limiting examples includes Opadry II Purple and Opadry II Yellow).

A third embodiment is directed to a composition according to the first embodiment or a unit dosage form according to the second embodiment for use in the treatment of hepatitis C virus infection in a human. In a first aspect of the third embodiment, the composition is used in combination with ribavirin.

In a second aspect of the third embodiment, the unit dosage form is used in combination with ribavirin.

### Tablet Preparation

The choice of particular types and amounts of excipients, and tabletting technique employed depends on the further properties of GS-7977 and the excipients, e.g., compressibility, flowability, particle size, compatibility, and density. In this regard, reference is made Remington: The Science and Practice of Pharmacy 2006, 21st edition, Lippincott Williams & Wilkins; *see also* Handbook of Pharmaceutical Excipients 1994, edited by A. Wade and P. J. Weller, The Pharmaceutical Press, 2nd Edition, London. A skilled formulation scientist may modify the formulations within the teachings of the specification to provide numerous formulations for a particular route of administration without rendering compositions containing GS-7977 unstable or compromising their therapeutic activity.

Tablets may be prepared according to methods known in the art, including dry granulation (e.g., roller compaction), wet granulation (e.g., fluid bed granulation and high shear granulation), and direct compression, and the type of excipients used will vary accordingly. It has been found that dry granulation is particularly suitable for providing high strength, low breakage tablets comprising relatively high concentrations of crystalline GS-7977 (e.g., about 33%), on a scale suitable for commercial production.

Suitable dry granulated tablets comprise granules comprising GS-7977 and one or more of a diluent, a disintegrant, a glidant, and a lubricant, wherein the granules are mixed with one or more of a diluent, a disintegrant, a glidant, and a lubricant to form a granulation mixture that is compressed to form tablets.

Also disclosed herein is a process for preparing a tablet composition comprising about 400 mg of GS-7977, said process comprising blending an intragranular composition and an extragranular composition to obtain a blended composition; compressing the blended composition to obtain a tablet composition; and optionally coating the tablet composition.

In a first example, the intragranular composition comprises GS-7977, a first intragranular diluent, optionally a second intragranular diluent, an intragranular disintegrant, an intragranular glidant, and an intragranular lubricant; and the extragranular composition comprises a first extragranular diluent, optionally a second extragranular diluent, an extragranular disintegrant, an extragranular glidant, and an extragranular lubricant, wherein the first intragranular diluent, the second intragranular diluent, the first extragranular diluent, and the second extragranular diluent are the same or different, the intragranular disintegrant and the extragranular disintegrant are the same or different, the intragranular glidant and the extragranular glidant are the same or different, and the intragranular lubricant and the extragranular lubricant are the same or different.

In a second example, the intragranular composition comprises GS-7977, a first intragranular diluent, an intragranular disintegrant, an intragranular glidant, and an intragranular lubricant; and the extragranular composition comprises a first extragranular diluent, a second extragranular diluent, an extragranular disintegrant, an extragranular glidant, and an extragranular lubricant, wherein the first intragranular diluent, the first extragranular diluent, and the second extragranular diluent are the same or different, the intragranular disintegrant and the extragranular disintegrant are the same or different, the intragranular glidant and the extragranular glidant are the same or different, and the intragranular lubricant and the extragranular lubricant are the same or different.

In a third example, the intragranular composition comprises GS-7977, a first intragranular diluent, a second intragranular diluent, an intragranular disintegrant, an intragranular glidant, and an intragranular lubricant; and the extragranular composition comprises a first extragranular diluent, an extragranular disintegrant, an extragranular glidant, and an extragranular lubricant, wherein the first intragranular diluent, the second intragranular diluent, and the first extragranular diluent are the same or different, the intragranular disintegrant and the extragranular disintegrant are the same or different, the intragranular glidant and the extragranular glidant are the same or different, and the intragranular lubricant and the extragranular lubricant are the same or different.

A fourth example of the fifth embodiment comprises at least one of the following steps:
(1) Sifting/Blending: GS-7977 and pharmaceutically acceptable excipients are sifted and/or blended during the formulation process. In one non-limiting example, first, GS-7977 and intragranular excipients (first diluent, optional second diluent, glidant, disintegrant; except for the intragranular lubricant) are sifted through a 20-mesh screen, added to a blender, and blended for a first blending time period to produce an initial blend. In one example, the first blending time period ranges from about 5 to about 30 minutes. Separately, an intragranular lubricant is passed through a 20-mesh screen, mixed with a portion of the initial blend, added to the blender, and blended for a second blending time period. In one example, the second blending time period is from about 1 minute to about 10 minutes. In another example, the second blending time period is from about 1 minute to about 5 minutes. In another example, the second blending time period is from about 5 minutes to about 10 minutes. Second, extragranular excipients (first diluent, optional second diluent, glidant, disintegrant) (except for the extragranular lubricant) are sifted through a 20-mesh screen and used in the final blending. It is contemplated that the blending time periods may increase as the scale of the formulation process increases.
(2) Dry Granulation:
   (A) Roller Compaction: GS-7977 and pharmaceutically acceptable excipients are passed through a roller compactor to product compacts. Compacts are then milled (below) to achieve granules. In one example, a blend comprising GS-7977, intragranular excipients, and lubricant, is passed through a roller compactor until granulation is achieved. The example has the following parameters: granulator speed ranges from about 50 to about 90 rpm, more specifically about 70 rpm; compactor speed ranges from about 4 to about 6 rpm, more specifically about 5 rpm; and pressure ranges from about 65 to about 100 barr, more specifically about 75 to about 100 bar.
   (B) Milling (preparation of milled/sifted granule): GS-7977 and pharmaceuticaly acceptable excipients are milled and/or sifted. In one example, after GS-7977 and intragranular excipients have passed through the roller compactor, the material is passed/forced through a 20-mesh screen using a Comill or Fitz Mill, and then sifted with a 60 mesh screen. In this example, material which remains on the 60 mesh screen is considered to be an acceptable granule, but material which passes through the 60 mesh screen is considered fines and is re-circulated through the roller compactor. This process is repeated until the percentage of fines is less than 20%. In one example, the mill speed ranges from about 50 to about 90 rpm, more specifically about 70 rpm,
(3) Final Blending: Granules comprising GS-7977 and intragranular excipients that have been milled/sifted are blended with extragranular excipients in a final blending. In one example, the milled/sifted granules comprising GS-7977 and intragranular exceipients are added to a blender (e.g., a double-cone blender, a bin blender, or a V-shell blender) along with extragranular excipients (first diluent and/or second diluent, glidant, and disintegrant) and blended for about 10 to about 30 minutes. The extragranular lubricant is passed through a 20-mesh screen and added to the blend. The blend/mixture is blended for about 5 minutes. It is contemplated that the blending time periods may increase as the scale of the formulation process increases.
(4) Compressing: The final blend is compressed into tablets using a tablet press (e.g., a Globe Pharma Mini Press).
(5) Optionally, tablets are film-coated with a film-coating agent.

In a fifth example, GS-7977 is blended with intragranular excipients comprising microcrystalline cellulose, mannitol, croscarmellose sodium and colloidal silicon dioxide in a blender. The mixture is milled and blended with a portion of magnesium stearate, then dry granulated using a roller compactor and mill. The resulting granules are then blended with extragranular excipients comprising microcrystalline cellulose, croscarmellose sodium, and colloidal silicon dioxide. An additional portion of magnesium stearate is added and the resulting composition is mixed to yield a powder blend comprising 33.33% w/w GS-7977. The powder blend is compressed into tablet cores to yield tablets comprising about 400 mg of GS-7977. The tablet cores are film-coated, and the resulting film-coated tablets are then packaged.

The embodiments described herein may be modified by one of ordinary skill without straying from the expressed intent using materials and methods described in Remington: The Science and Practice of Pharmacy 1995, edited by E. W. Martin, Mack Publishing Company, 19th edition, Easton, Pennsylvania; *see also* Handbook of Pharmaceutical Excipients 1994, edited by A. Wade and P. J. Weller, The Pharmaceutical Press, 2nd Edition, London. One of ordinary skill may modify the formulations within the teachings of the specification to provide numerous formulations without rendering compositions containing GS-7977 unstable or compromising its therapeutic activity. The following non-limiting examples provide further guidance related to additional aspects of the disclosed methods and compositions.

### Methods of Treatment

Also disclosed herein is a method for treating a subject infected with hepatitis C virus comprising administering to the subject for a time period an effective amount of GS-7977 and an effective amount of ribavirin.

In a first example, the time period is selected from among from about 2 weeks to about 12 weeks, from about 3 weeks to about 12 weeks, from about 4 weeks to about 12 weeks, from about 5 weeks to about 12 weeks, from about 6 weeks to about 12 weeks, from about 7 weeks to about 12 weeks, from about 8 weeks to about 12 weeks, from about 9 weeks to about 12 weeks, from about 10 weeks to about 12 weeks, from about 11 weeks to about 12 weeks, and about 12 weeks. In one example the time period is 12 weeks. In another example the time period is 8 weeks.

In a second example, the effective amount of GS-7977 is a daily dose selected from about 100 mg to about 800 mg, from about 200 mg to about 800 mg, from about 400 mg to about 800 mg, from about 600 mg to about 800 mg, from about 100 mg to about 600 mg, from about 100 mg to about 400 mg, from about 100 mg to about 200 mg, from about 200 mg to about 600 mg, from about 200 mg to about 400 mg, from about 400 mg to about 600 mg, and about 400 mg. In one example, the daily dose of GS-7977 is administered to the subject QD, BID, TID, or QID. In another example, a daily dose of about 400 mg of GS-7977 is administered to the subject QD, BID, TID, or QID. In another example, a daily dose of about 400 mg of GS-7977 is administered to the subject QD.

In a third example, an effective amount of GS-7977 is administered to the subject in combination with an effective amount of ribavirin, wherein the administration is concurrent or alternative.

In a fourth example, the effective amount of ribavirin is a daily dose selected from about 600 mg to about 1400 mg, and from about 800 mg to about 1200 mg. In one example, the effective amount of ribavirin is a daily dose of about 1000 mg to about 1200 mg. In another example, the effective amount of ribavirin is a daily dose of about 1000 mg to about 1200 mg based on the subject's body weight. In another example, the effective amount of ribavirin is a daily dose of about 800 mg. In another example, the daily dose of ribavirin is administered to the subject QD, BID, TID, or QID. In a further example, the daily dose of ribavirin is administered to the subject BID.

In a fifth example, a daily dose of about 400 mg of GS-7977 is administered to the subject in combination with a daily dose of about 800 mg to about 1200 mg of ribavirin. In one example, a daily dose of about 400 mg of GS-7977 is administered to the subject in combination with a daily dose of about 800 mg of ribavirin. In another example, a daily dose of about 400 mg of GS-7977 is administered to the subject in combination with a daily dose of about 1000 mg to about 1200 mg of ribavirin.

In a sixth example, the subject is infected with HCV genotype 1, 2, 3, 4, 5 or 6, or any combination thereof. In one example, the subject is infected with HCV genotype 1, 2, or 3, or any combination thereof.

In a seventh example, the subject has an undetectable amount of HCV RNA for at least 12 weeks after the end of the time period. In one example, the subject has an undetectable amount of HCV RNA for at least 24 weeks after the end of the time period. In another example, the subject has an undetectable amount of HCV RNA for at least 36 weeks after the end of the time period. In a further example, the subject has an undetectable amount of HCV RNA for at least 48 weeks after the end of the time period.

In an eighth example, the subject is a human.

In a ninth example, an effective amount of GS-7977 and an effective amount of ribavirin are administered to the subject according to an interferon-free treatment regimen. In one example, the interferon-free treatment regimen consists of administering an effective amount of GS-7977 and an effective amount of ribavirin to the subject for the time period.

In a tenth example, the effective amount of GS-7977 comprises a composition comprising GS-7977 and at least one pharmaceutically acceptable excipient as disclosed herein.

In an eleventh example, the effective amount of GS-7977 comprises a unit dosage form comprising GS-7977 and at least one pharmaceutically acceptable excipient as disclosed herein.

Further disclosed herein is a method of treating a subject infected with hepatitis C virus, said method comprising administering to the subject for a time period an effective amount of GS-7977 and an effective amount of ribavirin sufficient to produce an undetectable amount of HCV RNA in the subject for at least 12 weeks after the end of the time period.

In a first example, the time period is selected from among from about 2 weeks to about 12 weeks, from about 3 weeks to about 12 weeks, from about 4 weeks to about 12 weeks, from about 5 weeks to about 12 weeks, from about 6 weeks to about 12 weeks, from about 7 weeks to about 12 weeks, from about 8 weeks to about 12 weeks, from about 9 weeks to about 12 weeks, from about 10 weeks to about 12 weeks, from about 11 weeks to about 12 weeks, and about 12 weeks. In one example the time period is 12 weeks. In another example the time period is 8 weeks.

In a second example, the effective amount of GS-7977 is a daily dose selected from about 100 mg to about 800 mg, from about 200 mg to about 800 mg, from about 400 mg to about 800 mg, from about 600 mg to about 800 mg, from about 100 mg to about 600 mg, from about 100 mg to about 400 mg, from about 100 mg to about 200 mg, from about 200 mg to about 600 mg, from about 200 mg to about 400 mg, from about 400 mg to about 600 mg, and about 400 mg. In one example, the daily dose of GS-7977 is administered to the subject QD, BID, TID, or QID. In another example, a daily dose of about 400 mg of GS-7977 is administered to the subject QD, BID, TID, or QID. In another example, a daily dose of about 400 mg of GS-7977 is administered to the subject QD.

In a third example, an effective amount of GS-7977 is administered to the subject in combination with an effective amount of ribavirin, wherein the administration is concurrent or alternative.

In a fourth aspect of the seventh embodiment, the effective amount of ribavirin is a daily dose selected from about 600 mg to about 1400 mg, and from about 800 mg to about 1200 mg. In one example, the effective amount of ribavirin is a daily dose of about 1000 mg to about 1200 mg. In another example, the effective amount of ribavirin is a daily dose of about 1000 mg to about 1200 mg based on the subject's body weight. In another example, the effective amount of ribavirin is a daily dose of about 800 mg. In another example, the daily dose of ribavirin is administered to the subject QD, BID, TID, or QID. In a further example, the daily dose of ribavirin is administered to the subject BID.

In a fifth example, a daily dose of about 400 mg of GS-7977 is administered to the subject in combination with a daily dose of about 800 mg to about 1200 mg of ribavirin. In one example, a daily dose of about 400 mg of GS-7977 is administered to the subject in combination with a daily dose of about 800 mg of ribavirin. In another example, a daily dose of about 400 mg of GS-7977 is administered to the subject in combination with a daily dose of about 1000 mg to about 1200 mg of ribavirin.

In a sixth example, the subject is infected with HCV genotype 1, 2, 3, 4, 5 or 6, or any combination thereof. In one example, the subject is infected with HCV genotype 1, 2, 3, or any combination thereof.

In a seventh example, the subject has an undetectable amount of HCV RNA for at least 24 weeks after the end of the time period. In one example, the subject has an undetectable amount of HCV RNA for at least 36 weeks after the end of the time period. In another example, the subject has an undetectable amount of HCV RNA for at least 48 weeks after the end of the time period.

In an eighth example, the subject is a human.

In a ninth example, an effective amount of GS-7977 and an effective amount of ribavirin are administered to the subject according to an interferon-free treatment regimen. In one example, the interferon-free treatment regimen consists of administering an effective amount of GS-7977 and an effective amount of ribavirin to the subject for the time period.

In a tenth example, the effective amount of GS-7977 comprises a composition comprising GS-7977 and at least one pharmaceutically acceptable excipient as disclosed herein.

In an eleventh example, the effective amount of GS-7977 comprises a unit dosage form comprising GS-7977 and at least one pharmaceutically acceptable excipient as disclosed herein.

Further disclosed herein is a method of treating a human infected with hepatitis C virus, said method comprising administering to the human for a time period an effective amount of GS-7977 and an effective amount of ribavirin sufficient to produce an undetectable amount of HCV RNA in the human for at least 12 weeks after the end of the time period.

In a first example, the time period is selected from among from about 2 weeks to about 12 weeks, from about 3 weeks to about 12 weeks, from about 4 weeks to about 12 weeks, from about 5 weeks to about 12 weeks, from about 6 weeks to about 12 weeks, from about 7 weeks to about 12 weeks, from about 8 weeks to about 12 weeks, from about 9 weeks to about 12 weeks, from about 10 weeks to about 12 weeks, from about 11 weeks to about 12 weeks, and about 12 weeks. In one example the time period is 12 weeks. In another example the time period is 8 weeks.

In a second example, the effective amount of GS-7977 is a daily dose selected from about 100 mg to about 800 mg, from about 200 mg to about 800 mg, from about 400 mg to about 800 mg, from about 600 mg to about 800 mg, from about 100 mg to about 600 mg, from about 100 mg to about 400 mg, from about 100 mg to about 200 mg, from about 200 mg to about 600 mg, from about 200 mg to about 400 mg, from about 400 mg to about 600 mg, and about 400 mg. In one example, the daily dose of GS-7977 is administered to the human QD, BID, TID, or QID. In another example, a daily dose of about 400 mg of GS-7977 is administered to the human QD, BID, TID, or QID. In another example, a daily dose of about 400 mg of GS-7977 is administered to the human QD.

In a third example, an effective amount of GS-7977 is administered to the subject in combination with an effective amount of ribavirin, wherein the administration is concurrent or alternative.

In a fourth example, the effective amount of ribavirin is a daily dose selected from about 600 mg to about 1400 mg, and from about 800 mg to about 1200 mg. In one example, the effective amount of ribavirin is a daily dose of about 1000 mg to about 1200 mg. In another example, the effective amount of ribavirin is a daily dose of about 1000 mg to about 1200 mg based on the human's body weight. In another example, the effective amount of ribavirin is a daily dose of about 800 mg. In another example, the daily dose of ribavirin is administered to the human QD, BID, TID, or QID. In a further example, the daily dose of ribavirin is administered to the human BID.

In a fifth example, a daily dose of about 400 mg of GS-7977 is administered to the human in combination with a daily dose of about 800 mg to about 1200 mg of ribavirin. In one example, a daily dose of about 400 mg of GS-7977 is administered to the human in combination with a daily dose of about 800 mg of ribavirin. In another example, a daily dose of about 400 mg of GS-7977 is administered to the human in combination with a daily dose of about 1000 mg to about 1200 mg of ribavirin.

In a sixth example, the human is infected with HCV genotype 1, 2, 3, 4, 5, or 6, or any combination thereof. In one example, the subject is infected with HCV genotype 1, 2, or 3, or any combination thereof.

In a seventh example, the human has an undetectable amount of HCV RNA for at least 24 weeks after the end of the time period. In one example, the human has an undetectable amount of HCV RNA for at least 36 weeks after the end of the time period. In another example, the human has an undetectable amount of HCV RNA for at least 48 weeks after the end of the time period.

In an eighth example, an effective amount of GS-7977 and an effective amount of ribavirin are administered to the human according to an interferon-free treatment regimen. In one example, the interferon-free treatment regimen consists of administering an effective amount of GS-7977 and an effective amount of ribavirin to the subject for the time period.

In a ninth example, the effective amount of GS-7977 comprises a composition comprising GS-7977 and at least one pharmaceutically acceptable excipient as disclosed herein.

In a tenth example, the effective amount of GS-7977 comprises a unit dosage form comprising GS-7977 and at least one pharmaceutically acceptable excipient as disclosed herein.

Further disclosed herein is a method of treating a human infected with hepatitis C virus, said method comprising administering to the human for a time period an effective amount of GS-7977 and an effective amount of ribavirin sufficient to produce an amount of HCV RNA in the human that is less than about 15 IU/mL for at least 12 weeks after the end of the time period.

In a first example, the time period is selected from among from about 2 weeks to about 12 weeks, from about 3 weeks to about 12 weeks, from about 4 weeks to about 12 weeks, from about 5 weeks to about 12 weeks, from about 6 weeks to about 12 weeks, from about 7 weeks to about 12 weeks, from about 8 weeks to about 12 weeks, from about 9 weeks to about 12 weeks, from about 10 weeks to about 12 weeks, from about 11 weeks to about 12 weeks, and about 12 weeks. In one example the time period is about 12 weeks. In another example the time period is about 8 weeks.

In a second example, the effective amount of GS-7977 is a daily dose selected from about 100 mg to about 800 mg, from about 200 mg to about 800 mg, from about 400 mg to about 800 mg, from about 600 mg to about 800 mg, from about 100 mg to about 600 mg, from about 100 mg to about 400 mg, from about 100 mg to about 200 mg, from about 200 mg to about 600 mg, from about 200 mg to about 400 mg, from about 400 mg to about 600 mg, and about 400 mg. In one example, the daily dose of GS-7977 is administered to the human QD, BID, TID, or QID. In another example, a daily dose of about 400 mg of GS-7977 is administered to the human QD, BID, TID, or QID. In another example, a daily dose of about 400 mg of GS-7977 is administered to the human QD.

In a third example, an effective amount of GS-7977 is administered to the human in combination with an effective amount of ribavirin wherein the administration is concurrent or alternative.

In a fourth example, the effective amount of ribavirin is a daily dose selected from about 600 mg to about 1400 mg, and from about 800 mg to about 1200 mg. In one example, the effective amount of ribavirin is a daily dose of about 1000 mg to about 1200 mg. In another example, the effective amount of ribavirin is a daily dose of about 1000 mg to about 1200 mg based on the human's body weight. In another example, the effective amount of ribavirin is a daily dose of about 800 mg. In another example, the daily dose of ribavirin is administered to the human QD, BID, TID, or QID. In a further example, the daily dose of ribavirin is administered to the human BID.

In a fifth example, a daily dose of about 400 mg of GS-7977 is administered to the human in combination with a daily dose of about 800 mg to about 1200 mg of ribavirin. In one example, a daily dose of about 400 mg of GS-7977 is administered to the human in combination with a daily dose of about 800 mg of ribavirin. In another example, a daily dose of about 400 mg of GS-7977 is administered to the human in combination with a daily dose of about 1000 mg to about 1200 mg of ribavirin.

In a sixth example, the human is infected with HCV genotype 1, 2, 3, 4, 5, or 6, or any combination thereof. In one example, the human is infected with HCV genotype 1, 2, or 3, or any combination thereof.

In a seventh example, the human has an amount of HCV RNA less than about 15 IU/mL for at least 24 weeks after the end of the time period. In one example, the human has an amount of HCV RNA less than about 15 IU/mL for at least 36 weeks after the end of the time period. In another example, the human has an amount of HCV RNA less than about 15 IU/mL for at least 48 weeks after the end of the time period.

In an eighth example, an effective amount of GS-7977 and an effective amount of ribavirin are administered to the human according to an interferon-free treatment regimen. In one example, the interferon-free treatment regiment consists of administering an effective amount of GS-7977 and an effective amount of ribavirin to the subject for the time period.

In a ninth example, the effective amount of GS-7977 comprises a composition comprising GS-7977 and at least one pharmaceutically acceptable excipient as disclosed herein.

In a tenth example, the effective amount of GS-7977 comprises a unit dosage form comprising GS-7977 and at least one pharmaceutically acceptable excipient as disclosed herein.

Further disclosed herein is a method of treating a human infected with hepatitis C virus, said method consisting of administering to the human for a time period about 400 mg of GS-7977 and about 800 mg to about 1200 mg of ribavirin.

In a first example, the time period is selected from among from about 2 weeks to about 12 weeks, from about 3 weeks to about 12 weeks, from about 4 weeks to about 12 weeks, from about 5 weeks to about 12 weeks, from about 6 weeks to about 12 weeks, from about 7 weeks to about 12 weeks, from about 8 weeks to about 12 weeks, from about 9 weeks to about 12 weeks, from about 10 weeks to about 12 weeks, from about 11 weeks to about 12 weeks, and about 12 weeks. In one example the time period is 12 weeks. In another example the time period is 8 weeks.

In a second example, about 400 mg of GS-7977 is administered to the human daily. In one example, a daily dose of about 400 mg of GS-7977 is administered to the human QD, BID, TID, or QID. In another example, a daily dose of about 400 mg of GS-7977 is administered to the human QD.

In a third example, about 400 mg of GS-7977 is administered to the human in combination with about 800 mg to about 1200 mg of ribavirin, wherein the administration is concurrent or alternative.

In a fourth example, about 1000 mg to about 1200 mg of ribavirin is administered to the human daily. In one example, a daily dose of about 1000 mg to about 1200 mg of ribavirin is administered to the human QD, BID, TID, or QID. In another example, a daily dose of about 1000 mg to about 1200 mg of ribavirin is administered to the human BID. In a further example, a daily dose of 1000 mg or 1200 mg of ribavirin is administered to the subject based on body weight.

In a fifth example, about 800 mg of ribavirin is administered to the human daily. In one example, a daily dose of about 800 mg of ribavirin is administered to the human QD, BID, TD or QID. In another example, a daily dose of about 800 mg of ribavirin is administered to the human BID.

In a sixth example, the human is infected with HCV genotype 1, 2, 3, 4, 5 or 6, or any combination thereof. In one example, the human is infected with HCV genotype 1, 2, or 3, or any combination thereof.

In a seventh example, the human has an undetectable amount of HCV RNA for at least 12 weeks after the end of the time period. In one example, the human has an undetectable amount of HCV RNA for at least 24 weeks after the end of the time period. In another example, the human has an undetectable amount of HCV RNA for at least 36 weeks after the end of the time period. In a further example, the human has an undetectable amount of HCV RNA for at least 48 weeks after the end of the time period.

In an eighth example, the about 400 mg of GS-7977 comprises a composition comprising GS-7977 and at least one pharmaceutically acceptable excipient as disclosed herein.

In a ninth example, the about 400 mg of GS-7977 comprises a unit dosage form comprising GS-7977 and at least one pharmaceutically acceptable excipient as disclosed herein.

Further disclosed herein is a composition useful for the treatment of hepatitis C virus infection in a subject, said composition comprising an effective amount of GS-7977 and an effective amount of ribavirin.

In a first example, the composition does not comprise peginterferon.

In a second example, the effective amount of GS-7977 comprises from about 100 mg to about 800 mg, from about 200 mg to about 800 mg, from about 400 mg to about 800 mg, from about 600 mg to about 800 mg, from about 100 mg to about 600 mg, from about 100 mg to about 400 mg, from about 100 mg to about 200 mg, from about 200 mg to about 600 mg, from about 200 mg to about 400 mg, from about 400 mg to about 600 mg, and about 400 mg of GS-7977 administered to the subject daily. In one example, the composition comprises about 400 mg of GS-7977 administered to the subject QD.

In a third example, the effective amount of ribavirin comprises from about 600 mg to about 1400 mg, or from about 800 mg to about 1200 mg administered to the subject daily. In one example, the effective amount of ribavirin is about 1000 mg to about 1200 mg administered to the subject daily. In another example, the effective amount of ribavirin is about 1000 mg to about 1200 mg administered to the subject daily based on the subject's body weight. In another example, the effective amount of ribavirin about 800 mg administered to the subject daily. In another example, the composition comprises an effective amount ribavirin administered to the subject QD, BID, TID, or QID. In a further example, the composition comprises an effective amount of ribavirin administered to the subject BID.

In a fourth example, the composition comprises about 400 mg of GS-7977 administered to the subject QD and about 800 mg to about 1200 mg of ribavirin administered to the subject BID. In one example, the composition comprises about 400 mg of GS-7977 administered to the subject QD and about 800 mg of ribavirin administered to the subject BID. In another example, the composition comprises about 400 mg of GS-7977 administered to the subject QD and about 100 mg to about 1200 mg of ribavirin administered to the subject BID

In a fifth example, the composition is capable of providing an undetectable amount of HCV RNA for at least 12 weeks after the end of a time period following treatment of a subject infected with hepatitis C virus for the time period. In one example, the composition is capable of providing an undetectable amount of HCV RNA for at least 24 weeks after the end of a time period following treatment of a subject infected with hepatitis C virus for the time period. In another example, the composition is capable of providing an undetectable amount of HCV RNA for at least 36 weeks after the end of a time period following treatment of a subject infected with hepatitis C virus for the time period. In a further example, the composition is capable of providing an undetectable amount of HCV RNA for at least 48 weeks after the end of a time period following treatment of a subject infected with hepatitis C virus for the time period.

In a sixth example, the composition is capable of providing less than about 15 IU/mL of HCV RNA for at least 12 weeks after the end of a time period following treatment of a subject infected with hepatitis C virus for the time period. In one example, the composition is capable of providing less than about 15 IU/mL of HCV RNA for at least 24 weeks after the end of a time period following treatment of a subject infected with hepatitis C virus for the time period. In another example, the composition is capable of providing less than about 15 IU/mL of HCV RNA for at least 36 weeks after the end of a time period following treatment of a subject infected with hepatitis C virus for the time period. In a further example, the composition is capable of providing less than about 15 IU/mL of HCV RNA for at least 48 weeks after the end of a time period following treatment of a subject infected with hepatitis C virus for the time period.

In a seventh example, the effective amount of GS-7977 comprises a unit dosage form comprising GS-7977 and at least one pharmaceutically acceptable excipient as disclosed herein administered to the subject. In one example, the unit dosage form comprising GS-7977 and at least one pharmaceutically acceptable excipient as disclosed herein is administered to the subject QD.

Further disclosed herein is the to use of an effective amount of GS-7977 and an effective amount of ribavirin to treat hepatitis C virus infection in a subject in need thereof.

In a first example, the use comprises administering an effective amount of GS-7977 and an effective amount of ribavirin to the subject for a time period selected from among from about 2 weeks to about 12 weeks, from about 3 weeks to about 12 weeks, from about 4 weeks to about 12 weeks, from about 5 weeks to about 12 weeks, from about 6 weeks to about 12 weeks, from about 7 weeks to about 12 weeks, from about 8 weeks to about 12 weeks, from about 9 weeks to about 12 weeks, from about 10 weeks to about 12 weeks, from about 11 weeks to about 12 weeks, and about 12 weeks. In one example the time period is 12 weeks. In another example the time period is 8 weeks.

In a second example, the effective amount of GS-7977 is a daily dose selected from about 100 mg to about 800 mg, from about 200 mg to about 800 mg, from about 400 mg to about 800 mg, from about 600 mg to about 800 mg, from about 100 mg to about 600 mg, from about 100 mg to about 400 mg, from about 100 mg to about 200 mg, from about 200 mg to about 600 mg, from about 200 mg to about 400 mg, from about 400 mg to about 600 mg, and about 400 mg. In one example, the daily dose of GS-7977 is administered to the subject QD, BID, TID, or QID. In another example, a daily dose of about 400 mg of GS-7977 is administered to the subject QD, BID, TID, or QID. In another example, a daily dose of about 400 mg of GS-7977 is administered to the subject QD.

In a third example, an effective amount of GS-7977 is used in combination with an effective amount of ribavirin, wherein the administration of GS-7977 and ribavirin is concurrent or alternative.

In a fourth example, the effective amount of ribavirin is a daily dose selected from about 600 mg to about 1400 mg, and from about 800 mg to about 1200 mg. In one example, the effective amount of ribavirin is a daily dose of about 1000 mg to about 1200 mg. In another example, the effective amount of ribavirin is a daily dose of about 1000 mg to about 1200 mg based on the subject's body weight. In another example, the effective amount of ribavirin is a daily dose of about 800 mg. In another example, the daily dose of ribavirin is administered to the subject QD, BID, TID, or QID. In a further example, the daily dose of ribavirin is administered to the subject BID.

In a fifth example, the effective amount of GS-7977 is about 400 mg QD and the effective amount of ribavirin is about 800 mg to about 1200 mg BID. In one example, the effective amount of GS-7977 is about 400 mg QD and the effective amount of ribavirin is about 800 mg BID. In another example, the effective amount of GS-7977 is about 400 mg QD and the effective amount of ribavirin is about 1000 mg to about 1200 mg BID.

In a sixth example, the subject is infected with HCV genotype 1, 2, 3, 4, 5 or 6, or any combination thereof. In one example, the subject is infected with HCV genotype 1, 2, or 3, or any combination thereof.

In a seventh example, the subject has an undetectable amount of HCV RNA for at least 12 weeks after the end of the time period. In one example, the subject has an undetectable amount of HCV RNA for at least 24 weeks after the end of the time period. In another example, the subject has an undetectable amount of HCV RNA for at least 36 weeks after the end of the time period. In a further example, the subject has an undetectable amount of HCV RNA for at least 48 weeks after the end of the time period.

In an eighth example, the subject has an amount of HCV RNA less than about 15 IU/mL for at least 12 weeks after the end of the time period. In one example, the subject has an amount of HCV RNA less than about 15 IU/mL for at least 24 weeks after the end of the time period. In one example, the subject has an amount of HCV RNA less than about 15 IU/mL for at least 36 weeks after the end of the time period. In another example, the subject has an amount of HCV RNA less than about 15 IU/mL for at least 48 weeks after the end of the time period.

In a ninth example, the subject is a human.

In a tenth example, an effective amount of GS-7977 and an effective amount of ribavirin are used according to an interferon-free treatment regimen. In one example, the interferon-free treatment regimen consists of administering an effective amount of GS-7977 and an effective amount of ribavirin to the subject for a time period.

In an eleventh example, the effective amount of GS-7977 comprises a composition comprising GS-7977 and at least one pharmaceutically acceptable excipient as disclosed herein.

In a twelfth example, the effective amount of GS-7977 comprises a unit dosage form comprising GS-7977 and at least one pharmaceutically acceptable excipient as disclosed herein.

According to the FDA-approved label dated August 22, 2011, the recommended dose of COPEGUS® (ribavirin) tablets when used in combination with peginterferon depends on body weight and the HCV genotype to be treated, as shown in the following table.

| HCV Genotype | PEGASYS® Dose* | COPEGUS® Dose | Duration |
|---|---|---|---|
| Genotypes 1, 4 | 180 µg | <75 kg = 1000 mg | 48 weeks |
| | | ≥75 kg = 1200 mg | 48 weeks |
| Genotypes 2, 3 | 180 µg | 800 mg | 24 weeks |
| Genotypes 2 and 3 showed no increased response to treatment beyond 24 weeks. | | | |
| *See PEGASYS® Package Insert for further details on PEGASYS® dosing and administration. | | | |

The daily dose of COPEGUS® indicated for use in combination with peginterferon is 800 mg to 1200 mg administered orally in two divided doses (BID). The dose should be individualized to the subject depending on baseline disease characteristics (e.g., genotype), response to therapy, and tolerability of the regimen. Based on the foregoing, as well as the examples described below, an effective amount ribavirin when used in combination with an effective amount of GS-7977 is contemplated to include 800 mg and 1000 mg to 1200 mg, including daily doses of 1000 mg or 1200 mg depending on body weight.

Based on the data reported herein, an effective amount of GS-7977 is 400 mg QD, which can also be administered BID, TID, or QID. It is also contemplated that an effective amount of GS-7977 can include 100 mg to 400 mg and all integer values in between.

When administered as a combination, GS-7977 is administered to the subject in association with ribavirin. That is, the GS-7977 dose is administered during the same time period that the subject receives doses of ribavirin. Concurrent or alternative administration is considered, which means that while the GS-7977 and ribavirin are administered during the same time period, the specific order of administration on a daily basis can be: GS-7977 followed by ribavirin, GS-7977 and ribavirin together, or ribavirin followed by GS-7977. GS-7977 may be administered orally in capsule or tablet form, or any other suitable unit dosage form, in association with the oral (capsule or tablet form) administration of ribavirin. Of course, other types of administration of both medicaments, as they become available, are contemplated, such as by nasal spray, by a buccal or sublingual administration dosage form, transdermally, by suppository, by sustained release dosage form, etc. Any form of administration will work so long as the proper dosages are delivered without destroying the active ingredient and/or without impeding the effective amount of GS-7977 and/or an effective amount of ribavirin delivered to the subject.

### Examples

### GS-7977 Formulation Compositions Using Roller Compaction Process

A series of formulations containing polymorphic Form 1 GS-7977 with different quantitative compositions of excipients were prepared and screened using the roller compaction process to evaluate the impact of various diluents and compression aids on granulation powder properties and on tablet disintegration and dissolution times. Considerations were also given to moisture sorption properties of the tablets due to the sensitivity of Form 1 of GS-7977 to moisture.

All formulations were compressed into tablets at both high and low hardness levels. Formulation and tablet performance were determined by tablet disintegration time, content uniformity, and dissolution, as presented in **Table 1A.**

**Table 1A. Formulation Compositions for GS-7977 Form 1 Tablets Using a Roller Compaction Process**

| | **Formulation Composition (% w/w)** | | | | | | | | | | | |
|---|---|---|---|---|---|---|---|---|---|---|---|---|
| Ingredient | **A** | | **B1** | | **B2** | **C** | | **G1** | | **G2** | **H** | |
| Intragranular | | | | | | | | | | | | |
| GS-7977 | 25.0 | | 33.3 | | 33.3 | 33.3 | | 33.3 | | 33.3 | 33.3 | |
| Microcrystalline Cellulose | 25.0 | | 33.3 | | 33.3 | - | | - | | - | - | |
| Mannitol | - | | - | | - | 33.3 | | 30.6 | | 33.3 | 30.6 | |
| Croscarmellose Sodium | - | | - | | - | - | | 2.00 | | 3.0 | 2.00 | |
| Colloidal Silicon Dioxide | 0.5 | | 0.25 | | 0.3 | 0.25 | | 0.25 | | 0.3 | 0.25 | |
| Magnesium Stearate | - | | 0.5 | | 0.5 | 0.5 | | 0.5 | | 0.5 | 0.5 | |

| Extragranular | | | | | | | | | | | | |
|---|---|---|---|---|---|---|---|---|---|---|---|---|
| Microcrystalline Cellulose | 49.0 | | 31.9 | | 31.8 | - | | 15.3 | | 21.0 | - | |
| Mannitol | - | | - | | - | 31.9 | | 15.3 | | 5.8 | - | |
| Croscarmellose Sodium | - | | - | | - | - | | 2.0 | | 2.0 | 2.00 | |
| Dicalcium Phosphate | - | | - | | - | - | | - | | - | 30.6 | |
| Colloidal Silicon Dioxide | - | | 0.25 | | 0.25 | 0.25 | | 0.25 | | 0.3 | 0.25 | |
| Magnesium Stearate | 0.5 | | 0.5 | | 0.5 | 0.5 | | 0.5 | | 0.5 | 0.5 | |
| Total Tablet Weight (mg) | 400 | | 300 | | 300 | 300 | | 300 | | 300 | 300 | |
| Hardness (kp) Low/High | 8.1 | 16.3 | 7.4 | 17.2 | 10.2 | 5.8 | NA | 5.4 | 12.1 | 8.2 | 5.1 | 9.9 |
| Disintegration time (min:sec) | 0:17 | 0:33 | 0:13 | 3:16 | 0:48 | 45:00 | | 0:14 | 6:27 | 1:43 | 1:23 | 8:06 |
| Dissolution @ 45 min (% LS) | 98 | 102 | 94 | 91 | 82 | 87 | NA | 101 | 95 | 96 | 60 | 64 |

The results in **Table 1A** show that use of microcrystalline cellulose as the sole diluent (Formulations A, B1, B2) produced tablets with acceptable hardness, disintegration and dissolution, even without incorporating a disintegrant. In contrast, incorporation of mannitol as the sole diluent (Formulation C) without a disintegrant resulted in lower compressibility and a longer disintegration time resulting in slower dissolution. When used in combination with microcrystalline cellulose, mannitol levels as high as 75% of total filler amount (Formulation G) produced an acceptable tablet as long as a disintegrant was added to the formulation. However, lowering mannitol levels produced a harder and more robust tablet. Dicalcium phosphate used in combination with mannitol (Formulation H) failed to produce an acceptable tablet with respect to dissolution and hardness. The data in **Table 1A** support the use of formulations containing microcrystalline cellulose and mannitol/microcrystalline cellulose, in particular, as diluents.

Formulations B2 and G2 in **Table 1A** prepared per a roller compaction/granulation process were further evaluated. The prototype tablet batches were packaged 30 tablets per bottle and placed on stability in 40°C/75% RH conditions, with each bottle containing a molecular sieve (Tri-Sorb®) dessicant. The data shown in **Table 1B** show a decrease in moisture level as the amount of mannitol is increased (concomitant with reduction in microcrystalline cellulose).

**Table 1B. Stability Data for GS-7977 Form 1 Tablets Using a Roller Compaction Process**

| | | | **HPLC Assay** | | **Dissolution (% Dissolved)^{a}** | | | | |
|---|---|---|---|---|---|---|---|---|---|
| **Formulation** | **Stability Condition** | **Time (mo.)** | **% LS** | **Unknown % img/deg at RRT 0.67** | **15 min** | **30 min** | **45 min** | **60 min** | **Water Content (%)** |
| **B2** | 25 °C/60% RH | 0 | 98.5 | - | 76 (4) | 80 (3) | 82 (3) | 85 (3) | 4.3 |
| | | 3 | 98.4 | - | 73 (2) | 77 (3) | 79 (3) | 81 (3) | 3.2 |
| | | 6 | 96.2 | <0.04 | 76 (2) | 80 (2) | 82 (2) | 85 (2) | 3.2 |
| | 40 °C/75% RH | 3 | 97.0 | - | 71 (5) | 74 (5) | 77 (4) | 79 (4) | 3.3 |
| | | 6 | 96.8 | 0.04 | 79 (8) | 84 (8) | 87 (8) | 89 (7) | 3.5 |
| **G2** | 25 °C/60% RH | 0 | 99.9 | - | 84 (6) | 93 (3) | 96 (3) | 98 (3) | 1.7 |
| | | 3 | 98.3 | <0.04 | 72 (8) | 92 (2) | 96 (2) | 97 (3) | 1.4 |
| | | 6 | 97.9 | <0.04 | 82 (4) | 93 (2) | 95 (3) | 97 (2) | 1.3 |
| | 40 °C/75% RH | 3 | 98.0 | <0.04 | 77 (4) | 84 (4) | 88 (3) | 89 (3) | 1.5 |
| | | 6 | 99.0 | <0.04 | 80 (5) | 90 (5) | 93 (4) | 94 (4) | 1.2 |
| | 40 °C/75% RH (no dessicant) | 3 | 97.9 | 0.08 | 79 (4) | 91 (3) | 96 (2) | 97 (2) | 1.9 |
| | | 6 | 97.8 | 0.18 | 72 (8) | 85 (2) | 91 (2) | 93 (3) | 1.9 |
| ^{a}Dissolution method: USP Apparatus II (paddles) with 900 mL, pH 6.8 (50 mM sodium phosphate), 0.5% SLS, 75 rpm, 37 °C | | | | | | | | | |

### GS-7977 400 mg Tablets

Formulations (**Tablets A** and **B**) comprising GS-7977 polymorphic Form 1 were prepared by dry granulation. The formulations contained GS-7977 (polymorphic Form 1) (33.33%), mannitol (30.00%), microcrystalline cellulose (29.67%), croscarmellose sodium (5.00%), colloidal silicon dioxide (0.50%), and magnesium stearate (1.50%), as described in **Table 2.**

**Table 2. GS-7977 Polymorphic Form 1 400 mg Tablet Compositions**

| | **% w/w of 400 mg tablet** | |
|---|---|---|
| | **Tablet A** | **Tablet B** |
| **Intragranular Components** | | |
| GS-7977 (Form 1) | 33.33 | 33.33 |
| Mannitol | 30.0 | 30.0 |
| Croscarmellose Sodium | 3.0 | 3.0 |
| FD&C Red 40 Aluminum Lake | -- | 0.27 |
| FD&C Blue 2 Aluminum Lake | -- | 0.10 |
| Colloidal Silicon Dioxide | 0.25 | 0.25 |
| Magnesium Stearate | 0.50 | 0.5 |

| **Extragranular Components** | | |
|---|---|---|
| Microcrystalline Cellulose | 29.67 | 29.12 |
| Croscarmellose Sodium | 2.00 | 2.00 |
| FD&C Red 40 Aluminum Lake | -- | 0.13 |
| FD&C Blue 2 Aluminum Lake | -- | 0.05 |
| Colloidal Silicon Dioxide | 0.25 | 0.25 |
| Magnesium Stearate | 1.00 | 1.00 |
| **Total** | 100.00 | 100.00 |
| Coating Agent | 3.00 | -- |

Tablets containing about 400 mg of GS-7977 (Form 1) per tablet and an Opadry II purple film coating (**Tablet A** in **Table 2**) were prepared as follows:
(1) A composition comprising GS-7977 (Form 1) and the intragranular excipients (mannitol, croscarmellose sodium, and colloidal silicon dioxide) was sifted through a 20-mesh screen and added to a blender (V-shell blender) and blended for about 10-15 minutes to obtain an initial blend. Separately, the intragranular magnesium stearate was passed through a 20-mesh screen and mixed with a portion of the initial blend, added to the blender, and blended for about 5 minutes to obtain an intragranular blend.
(2) Separately, the extragranular excipients microcrystalline cellulose, croscarmellose sodium, and colloidal silicon dioxide were sifted through a 20-mesh screen for use in the final blending (step (4), below).
(3) The intragranular blend comprising GS-7977, mannitol, croscarmellose sodium, colloidal silicon dioxide, and magnesium stearate was passed through a roller compactor equipped with a 20-mesh (0.84 mm) milling screen on the granulator and both 20- and 60-mesh (0.25 mm) screens on the separator until granulation was achieved. The roller compactor parameters were: (i) granulator speed ranges from about 50 to about 90 rpm, more specifically about 70 rpm, compactor speed ranges from about 4 to about 6 rpm, more specifically about 5 rpm, and pressure ranges from about 65 to about 100 barr, more specifically about 75 to about 100 bar. Ribbons were produced using flat straight-grooved rollers. Upon passing through the roller compactor, the material was passed/forced through a 20-mesh screen and then sifted with a 60 mesh screen. Granules were sorted into three categories (coarse, acceptable, and fine) using the separator portion of the dry granulator. 'Coarse' granules retained on the 20-mesh (0.84 mm) screen on the separator were passed through a Comil with a 0.055-inch (1.4 mm) round screen. Granules that remained on the 60 mesh screen were considered to be 'acceptable' granules. The milled/sifted granule material was passed to the final blending step. Material that passed through the 60 mesh screen was considered 'fine' and was re-circulated through the roller compactor. This process was repeated until a minimum amount (e.g., less than 20%) of fines remained.
(4) The milled/sifted granules from step (3) and the sifted extragranular excipients (microcrystalline cellulose, mannitol, croscarmellose sodium and silicon dioxide) from step (2) were added to a blender (V-shell blender) and blended for about 15 minutes. Separately, magnesium stearate was passed through a 20-mesh screen. The magnesium stearate was added to the blender and blended for about 5 minutes to obtain a final powder blend comprising 33.33% w/w GS-7977. Blend uniformity samples were taken prior to removing the blend from the blender.
(5) The final blend was compressed into tablets using a tablet press (e.g., a Globe Pharma Mini Press) to obtain 1200 mg uncoated tablets comprising about 400 mg of GS-7977. As needed, a 15% w/w aqueous suspension for film-coating comprising polyvinyl alcohol (Opadry II Purple) was prepared and applied to achieve a target weight gain of 3% (range: 2-4%). The coating suspension was sprayed at 300 g/min/4 guns (range: 200-400 g/min/4 guns) at a target pan speed of 5 rpm (range: 4-8 rpm) and an exhaust temperature of 46 ±5 °C. The GS-7977 tablets were packaged with 30 tablets and 1 gram of desiccant per bottle.

Uncoated tablets comprising 400 mg of GS-7977 (Form 1) were prepared in a similar manner using blue and red lake in the blend **(Tablet B** in **Table 2**).

Another formulation **(Tablet C**) was prepared containing GS-7977 polymorphic Form 6 (33.33%), mannitol (30.00%), microcrystalline cellulose (29.67%), croscarmellose sodium (5.00%), colloidal silicon dioxide (0.50%), and magnesium stearate (1.50%), as described in **Table 3.** While a low moisture grade of microcrystalline cellulose (PH 112) was used in the Form 1 formulation to improve the stability of Form 1 GS-7977, the microcrystalline cellulose grade was changed to PH 102 for the **Tablet C** formulation due to the non-hygroscopic nature of Form 6. In addition, incorporation of a large proportion of the excipients into the intragranular composition decreased the potential for powder segregation and the variability in the blend, and improved the tablet content uniformity for the Tablet C formulation.

**Table 3. GS-7977 Polymorphic Form 6 400 mg Tablet Composition**

| | **Tablet C** | |
|---|---|---|
| | **% w/w** | **mg/tablet** |
| **Intragranular Components** | | |
| GS-7977 (Form 6) | 33.33 | 400.0 |
| Mannitol | 30.00 | 360.0 |
| Microcrystalline Cellulose | 24.67 | 296.0 |
| Croscarmellose Sodium | 2.50 | 30.0 |
| Colloidal Silicon Dioxide | 0.45 | 5.4 |
| Magnesium Stearate | 0.75 | 9.0 |

| **Extragranular Components** | | |
|---|---|---|
| Microcrystalline Cellulose | 5.00 | 60.0 |
| Croscarmellose Sodium | 2.50 | 30.0 |
| Colloidal Silicon Dioxide | 0.05 | 0.6 |
| Magnesium Stearate | 0.75 | 9.0 |
| **Total** | 100.00 | 1200 |
| Coating Agent | 3.0 | 36.0 |

The **Tablet C** formulation was prepared by blending the intragranular components listed in **Table 3**, other than magnesium stearate (i.e., GS-7977, microcrystalline cellulose, mannitol, croscarmellose sodium and colloidal silicon dioxide) in a blender. The mixture was milled, blended with the intragranular magnesium stearate and dry granulated using a roller compaction process train and mill. The resulting ribbons were milled through a milling screen and then blended with the extragranular excipients (microcrystalline cellulose, croscarmellose sodium, colloidal silicon dioxiade, magnesium stearate) to yield a powder blend comprising 33.33% w/w GS-7977. The powder blend was compressed to a target tablet weight of 1200 mg, with each tablet comprising about 400 mg of GS-7977. An aqueous suspension for the film-coating process was prepared and applied to achieve a target weight gain of 3%.

Moisture content was tested for **Tablets A-C** and tablet stability (30 tablets/bottle with a 1 gram Tri-sorb® dessicant in a 60 cc HDPE bottle) was tested for **Tablets B** and **C**, the results of which are presented in **Table 4.**

**Table 4. Moisture and Stability Data for GS-7977 400 mg Tablets**

| | **Tablet A** | | | **Tablet B** | | | **Tablet C** |
|---|---|---|---|---|---|---|---|
| | **Moisture (% w/w)** | **HPLC Assay** | | **Moisture (% w/w)** | **HPLC Assay** | | **HPLC Assay** |
| | | **% GS-7977** | **% Impurity** | | **% GS-7977** | **% Impurity** | **% GS-7977** |
| Initial | 1.8 | 100 | 0.05 | 1.5 | 99.5 | 0.05 | 101.7 |
| 40°C/75% RH | | | | | | | |
| 1 month | 1.4 | 102.4 | 0.04 | 1.5 | 101.7 | 0.05 | 101.1 |
| 2 months | 1.5 | 101.5 | 0.04 | 1.7 | 101.1 | 0.04 | 100.9 |

The results in **Table 4** show that the exemplary tablet compositions described herein exhibit stability to both moisture and degradation.

The dissolution profile (75 RPM, Apparatus II (Paddle), Phosphate buffer pH 6.8 900 mL) of tablets having the **Tablet B** formulation was tested initially and after storage at 40°C and 75% relative humidity. The results are presented in **Table 5.**

**Table 5. Dissolution Data for GS-7977 (Form 1) 400 mg Tablet B Composition**

| | **Mean Dissolution (± RSD^{a})** | | | |
|---|---|---|---|---|
| | **15 min** | **30 min** | **45 min** | **60 min** |
| Initial | 97 ± 1 | 102 ± 2 | 103 ± 1 | 102 ± 1 |
| 40°C/75% RH | | | | |
| 1 month | 87 ± 3 | 99 ± 2 | 101 ± 2 | 100 ± 3 |
| 2 month | 96 ± 1 | 102 ± 1 | 102 ± 1 | 102 ± 1 |

| | | | | |
|---|---|---|---|---|
| ^{a}RSD = Relative Standard Deviation | | | | |

### In Vitro Antiviral Synergy for the Combination of GS-977 and Ribavirin

The antiviral effect of GS-7977 in combination with ribavirin was evaluated using the HCV genotype 1a replicon. (Robinson et al., Antimicrob. Agents Chemother. (2010) 54(8): 3099-3106.) The cells were grown in cell culture medium containing Dulbecco's Modified Eagle Medium (DMEM) with Gibco® GlutaMAX supplemented with 10% HyClone FBS, 100 units/mL penicillin, 100 µg/mL streptomycin, and 0.1 mM non-essential amino acids. Replicon cells were maintained in 0.5 mg/mL Geneticin®. The cells were passaged every 3-4 days before reaching confluency. All compounds were supplied in 100% DMSO and compound serial dilutions were performed in 100% DMSO. To each well of a 384-well plate was added 90 µL of cell culture medium (without Geneticin®) containing 2000 suspended HCV replicon cells and 0.4 µL of compound solution. The DMSO concentration of the final assay wells was 0.44%. The plates were incubated for 3 days at 37°C with 5% CO₂ and 85% humidity.

For the CC₅₀ assay, the media in the 384-well plate was aspirated and the wells were washed four times with 100 µL 1 X PBS each. A volume of 50 µL of a solution containing 400 nM calcein AM in 1 X PBS was added to each well and the plate was incubated for 30 minutes at room temperature before the fluorescence signal (excitation 490 nm, emission 520 nm) was measured.

EC₅₀ assays were performed in the same wells as CC₅₀ assays. The calcein-PBS solution was aspirated and a volume of 20 µL of Dual-Glo® luciferase buffer was added to each well. The plate was incubated for 10 minutes at room temperature and a volume of 20 µL of a solution containing a 1:100 mixture of Dual-Glo® Stop & Glo® substrate and Dual-Glo® Stop & Glo® buffer was added to each well. The plate was incubated at room temperature for 10 minutes before the luminescence signal was measured.

The combination study experimental data were analyzed for two-compound synergy using the MacSynergy II program developed by Prichard and Shipman. (Prichard et al., MacSyngergy™ II, Version 1.0, University of Michigan, Ann Arbor (1993).) Two-compound synergy definitions are provided in **Table 6**:

**Table 6. Two-Compound Synergy Definitions**

| Synergy/Antagonism Volume (nM²%) | Interaction |
|---|---|
| >100 | Strong Synergy |
| >50 and ≤ 100 | Moderate Synergy |
| > 25 and ≤ 50 | Minor Synergy |
| ≤ 25 and > -25 | Additive |
| ≤ -25 and > -50 | Minor Antagonism |
| ≤ -50 and > -100 | Moderate Antagonsim |
| ≤ -100 | Strong Antagonsim |

GS-7977 in combination with ribavirin showed a synergy volume of 35.3 ± 3.2 nM²% indicating a synergistic interaction. A cytotoxicity study analyzing the combined effect of GS-7977 and ribavirin showed cell viability greater than 85% at the highest combined drug concentrations (320 nM GS-7977, 1600 nM ribavirin, 14.0 ± 4.4 % inhibition on cell growth). (*See also* Hebner et al., 63rd Annual Meeting of the American Association for the Study of Liver Diseases, Poster 1875, Nov. 12, 2012.) These findings support the potential of GS-7977 administered in combination with ribavirin to achieve enhanced viral suppression compared to GS-7977 or ribavirin monotherapy.

### In Vitro Susceptibility of S282T Mutants to GS-7977, Ribavirin, and the Combination of GS-7977 and Ribavirin

*In vitro* studies have shown that S282T is the primary mutation selected by GS-7977 in HCV genotype 1a, 1b and2areplicon cells. (Lam et al., J. Virology (2011) 85(23): 12334-12342; Lam et al., Antimicrob. Agents Chemother. (2012) 56(6): 3359-3368.) S282T mutations in NS5B were created by site-directed mutagenesis in 1a-H77, 1b con-1, and 2a JFH1 sub-genomic replicons. 1b con-1-based chimeric replicons containing 2b, 3a, 4a, 5a, or 6a NS5B were also engineered to harbor the S282T mutation. (*See* Wong et al., Virology (2012) 429:57-62.) Replication capacities and drug susceptibilities of S282T to GS-7977 and ribavirin were determined in transient replicon assays. The susceptibilities of S282T and wild-type (WT) NS5B to GS-7977 and ribavirin were further studied by passaging the mixture of 50% S282T and 50% WT in GT2a in the presence of GS-7977 and ribavirin individually and in combination. Relative percentages of mutant and WT were assessed by deep sequencing.

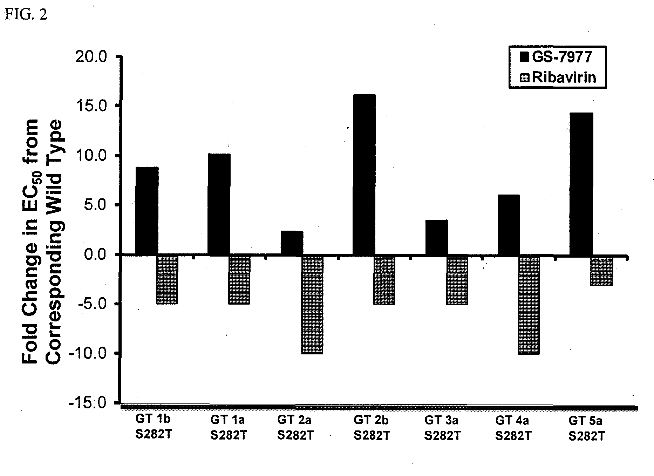

Introduction of the NS5B S282T mutation into 1b, 1a, 2a, 2b, 3a, 4a, and 5a HCV replicons resulted in reduced susceptibility to GS-7977 for all seven genotypes, producing a 2- to 16-fold increase in EC₅₀ values compared to the wild-type from the corresponding genotypes. Surprisingly, the S282T replicons were 3- to 10-fold more sensitive to treatment with ribavirin than their corresponding wild-type for these seven genotypes. EC₅₀ values were not calculated for genotype 6a S282T mutants due to low signal-to-noise ratios; the genotype 6a mutant did not replicate sufficiently to obtain drug susceptibility data. The results of these studies are presented in **Table 7**, below, and in Figure 2.

**Table 7. Antiviral Activity of GS-7977 and Ribavirin Against S282T Mutants in Genotype 1-6 Replicons**

| Genotype | GS-7977 | | | Ribavirin | | |
|---|---|---|---|---|---|---|
| | EC₅₀ nM^{a} | | Fold Change^{b} | EC₅₀ nM^{a} | | Fold^{b} Change |
| | WT | S282T | | WT | S282T | |
| 1b | 21.5 | 189.2 | 8.8 | 6.6 | 1.6 | 0.2 |
| 1a | 25.1 | 253.1 | 10.1 | 21.0 | 5.0 | 0.2 |
| 2a | 146.8 | 346.1 | 2.4 | 8.3 | 0.6 | 0.1 |
| 2b^{c} | 13.3 | 215.6 | 16.2 | 2.6 | 0.6 | 0.2 |
| 3a^{c} | 33.9 | 117.1 | 3.5 | 6.7 | 1.0 | 0.2 |
| 4a^{c} | 35.8 | 217.5 | 6.1 | 6.2 | 0.6 | 0.1 |
| 5a^{c} | 9.91 | 142.2 | 14.35 | 1.9 | 0.6 | 0.3 |
| 6a^{c} | 39.8 | n/a^{d} | - | 5.3 | n/a^{d} | - |
| ^{a}EC₅₀ indicates average of 2 or more independent experiments. | | | | | | |
| ^{b}Fold change from corresponding wild-type. | | | | | | |
| ^{c}These chimeric replicons carry NS5B from genotypes 2b, 3a, 4a; however, the NS5A sequence in all of these chimeric replicons is derived from genotype 1b. | | | | | | |
| ^{d}EC₅₀ was not determined due to low signal-to-noise ratio. | | | | | | |

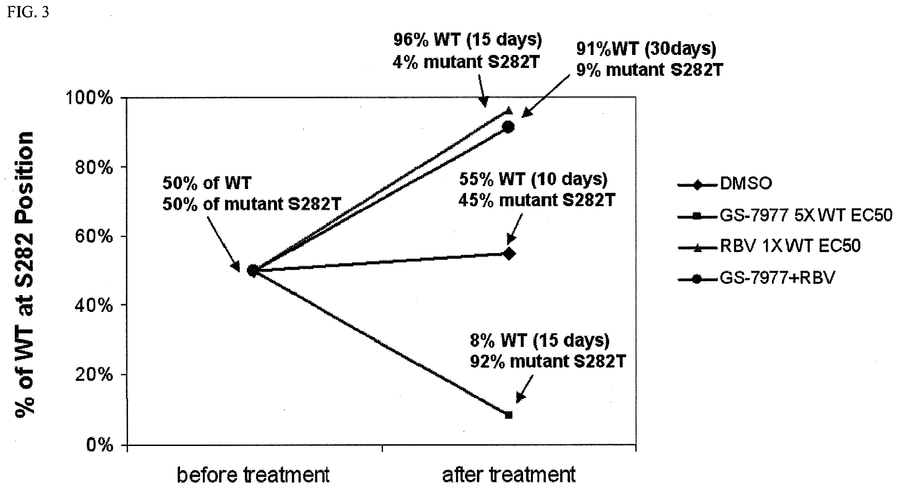

A long-term passaging study in GT 2a replicons revealed that GS-7977 alone displayed greater inhibition of WT than S282T, resulting in a population that was 92% mutant S282T over fifteen days. Ribavirin alone suppressed S282T more than WT, resulting in a population that was 96% WT after fifteen days. The combination of GS-7977 and ribavirin also preferentially inhibited S282T over WT, resulting in a population that was 91% WT following thirty days of treatment. The results of the passaging study are presented in Figure 3. (*See also* Han et al., 63rd Annual Meeting of the American Association for the Study of Liver Diseases, Poster 1078, Nov. 11, 2012.)

Thus, while the S282T replicon has been shown to confer reduced susceptibility to GS-7977 *in vitro*, the mutant replicon has demonstrated increased susceptibility to ribavirin over the wile-type, suggesting that treatment of CHC with the combination of GS-7977 and ribavirin may result in reduced viral breakthroughs and incidence of resistance compared to monotherapy with GS-7977 alone. The hypersensitivity of S282T mutants to ribavirin may provide an additional advantage to combination treatment comprising GS-7977 and ribavirin, in terms of preventing or delaying the emergency of S282T mutants.

### Quantification of HCV RNA in Human Clinical Studies

Quantitative HCV RNA testing for clinical trials was performed using the Roche COBAS® AmpliPrep/COBAS® HCV TaqMan® assay using a standardized, automatic RNA extraction system and standardized controls and calibrators. The established LOD of the assay was 15 IU/mL (defined by a 95% hit rate with WHO Standards). HCV RNA levels were measured using serum samples.

US 2010/0226885 (US 12/376,180) also discloses a method for measuring whether a patient has achieved an HCV negative status using RT-PCR to measure HCV RNA levels.

### Treatment Regimens - P7977-0221 and PROTON Clinical Studies

A Phase 2a, 3-cohort placebo-controlled study (P7977-0221) evaluated treatment with GS-7977 (100 mg, 200 mg or 400 mg QD) in combination with peginterferon and ribavirin in treatment-naive GT1 HCV subjects for 4 weeks, followed by up to an additional 44 weeks of treatment with SOC peginterferon and ribavirin. High RVR (88-94%) was observed for all three GS-7977 treatment groups. Following discontinuation of GS-7977, the durability of antiviral response (SVR-12 and SVR-24) was greatest in the 400 mg treatment group (86.7% and 80.0%, respectively). SVR-12 and SVR-24 rates were 72.2% and 83.3%, respectively, for patients receiving a 200 mg GS-7977 treatment regimen, and the majority of GS-7977-treated patients who failed to achieve SVR received a 100 mg QD dose of GS-7977.

The Phase 2b PROTON study evaluated treatment with a combination of GS-7977, peginterferon, and ribavirin at daily dosage levels of 200 mg and 400 mg of GS-7977 for 12 weeks, followed by up to an additional 36 weeks of treatment with SOC peginterferon and ribavirin. A greater number of subjects experienced viral breakthrough after cessation of the GS-7977 200 mg dosage level while still receiving peginterferon/ribavirin treatment compared to no viral breakthroughs after cessation of the GS-7977 400 mg dosage level while still receiving peginterferon/ribavirin treatment.

The preceding studies indicate enhanced efficacy for a GS-7977 400 mg daily dose level compared to a 200 mg daily dose level.

### Treatment Regimens - ELECTRON Clinical Study

The ongoing Phase 2a ELECTRON clinical study evaluated GS-7977 400 mg QD for 8 or 12 weeks in combination with or without ribavirin and/or peginterferon in subjects with GT1, GT2 or GT3 HCV infection. Preliminary data demonstrates 100% SVR-12 for treatment-naive GT2 or GT3 HCV patients treated with a combination of GS-7977 and ribavirin, regardless of the presence of peginterferon, as well as 84% SVR-12 for treatment-naive GT1 HCV patients receiving combination treatment with GS-7977 and ribavirin. In comparison, only 60% of treatment-naive GT2/GT3 HCV patients receiving GS-7977 monotherapy achieved SVR-12.

Part 1 of the ELECTRON trial evaluated 12-week regimens of GS-7977 400 mg QD in combination with ribavirin (RBV) only (1000/12000 mg by weight BID) and, in separate arms, with abbreviated durations of peginterferon for 4, 8, or 12 weeks in treatment-naive patients with HCV GT2 or GT3:
Group 1: GS-7977 (400 mg QD) with RBV (1000/1200 mg BID) for 12 weeks (no peginterferon) (GT2/GT3 treatment-naive); and
Groups 2, 3, 4: GS-7977 (400 mg QD) with RBV (1000/1200 mg BID) for 12 weeks and PEG (180 µg weekly) weeks 1-4 only / PEG (180 µg weekly) weeks 1-8 only / PEG (180 µg weekly) weeks 1-12 (GT2/GT3 treatment-naive).

In Part 2 of the ELECTRON trial, an additional 30 patients were enrolled in exploratory regimens of GS-7977 monotherapy and abbreviated durations of total therapy with the combination of GS-7977, RBV and PEG:
Group 5: GS-7977 (400 mg QD) monotherapy for 12 weeks (GT2/GT3 treatment-naïve);
Group 6: GS-7977 (400 mg QD) with PEG (180 µg weekly) and RBV (1000/1200 mg BID) for 8 weeks (GT2/GT3 treatment-naive); and
Group 7: GS-7977 (400 mg QD) with RBV (1000/1200 mg BID) for 12 weeks (GT1 null responders).

In Part 3 of the ELECTRON trial, two additional peginterferon-free regimens were explored in treatment-naive patients with HCV GT1 and treatment-experienced patients with HCV GT2 or HCV GT3:
Group 8: GS-7977 (400 mg QD) with RBV (1000/1200 mg BID) for 12 weeks (GT1 treatment-naive); and
Group 9: GS-7977 (400 mg QD) with RBV (1000/1200 mg BID) for 12 weeks (GT2/GT3 treatment-experienced).

In Part 4 of the ELECTRON trial, two further peginterferon-free regimens were added:
Group 10: GS-7977 (400 mg QD) with RBV (1000/1200 mg BID) for 8 weeks (GT2/GT3 treatment-naive); and
Group 11: GS-7977 (400 mg QD) with RBV (800 mg BID) for 12 weeks (GT2/GT3 treatment-naive).

Null responders were defined as patients with <2 log₁₀ IU/mL decline from baseline HCV RNA after at least 12 weeks of treatment with peginterferon and ribavirin.

Treatment-experienced patients were defined as those who had any of the following responses after at least 12 weeks of treatment with peginterferon and ribavirin: (1) < 2 log₁₀ IU/mL decline from baseline HCV RNA, (2) ≥ log₁₀ IU/mL reduction in HCV RNA, but HCV RNA > limit of quantitation ("LOQ") at end of treatment, and (3) HCV RNA < LOQ at end of treatment but subsequent HCV RNA > LOQ (relapsers).

The preliminary results of the ELECTRON trial are presented below.

The patient population and demographics for ELECTRON Groups 1-9 are summarized in **Tables 8A** and **8B**, below.

**Table 8A. ELECTRON Patient Demographics (Groups 1-5)**

| | GS-7977 RBV NO PEG | GS-7977 RBV 4 Wks PEG | GS-7977 RBV 8 Wks PEG | GS-7977 RBV 12 Wks PEG | GS-7977 NO RBV NO PEG |
|---|---|---|---|---|---|
| | GT2/GT3 Tx-Naive | | | | |
| | (Group 1) | (Groups 2, 3, 4) | | | (Group 5) |
| Number (N) | 10 | 9 | 10 | 11 | 10 |
| Male (n, %) | 8 (80) | 5 (56) | 5 (50) | 9 (82) | 4 (40) |
| Race (Caucasian, %) | 7 (70) | 4 (44) | 8 (80) | 8 (73) | 7 (70) |
| Age (Mean, range) | 47 (35-53) | 47 (29-66) | 49 (29-66) | 46 (22-57) | 43 (22-57) |
| BMI (Mean, range) (kg/m²) | 28 (23.7-35.7) | 26 (21.3-32.2) | 25 (18.1-32.5) | 24 (20.8-28.4) | 26 (18.2-39.4) |
| HCV RNA (Mean, SD) (log₁₀ IU/mL) | 6.7 (0.42) | 6.6 (0.52) | 6.4 (0.57) | 6.3 (0.76) | 5.7 (0.89) |
| HCV RNA (Median, range) | 6.7 (6.6-7.3) | 6.6 (5.8-7.3) | 6.4 (5.1-7.0) | 6.4 (5.2-7.1) | 5.7 (4.6-7.3) |
| HCV GT-2:GT-3 | 4:6 | 3:6 | 4:6 | 4:7 | 3:7 |
| IL28B CC/CT/TT | 5/4/1 | 4/4/1 | 4/4/2 | 4/5/2 | 2/6/2 |
| IL28B CC (n, %) | 5 (50) | 4 (44) | 4 (40) | 4 (36) | 2 (20) |

**Table 8B. ELECTRON Patient Demographics (Groups 6-9)**

| | GS-7977 RBV PEG 8 Wks GT2/GT3 Tx-Naive | GS-7977 RBV NO PEG 12 Wks GT1 Null | GS-7977 RBV NO PEG 12 Wks GT1 Tx-Naive | GS-7977 RBV NO PEG 12 Wks GT2/GT3 Tx-Experienced |
|---|---|---|---|---|
| | (Group 6) | (Group 7) | (Group 8) | (Group 9) |
| Number (N) | 10 | 10 | 25 | 25 |
| Male (n, %) | 50 | 70 | 60 | 76 |
| Race (Caucasian, %) | 70 | 90 | 80 | 68 |
| BMI (Mean, range) | 24.8 (21-34.9) | 28.1 (19.5-35.7) | 25.6 (19.3-37.6) | 26.8 (19.2-40.0) |
| HCV RNA (Mean, SD) (log₁₀ IU/mL) | 6.1 (4.3-7.3) | 6.8 (5.6-7.5) | 6.1 (4.4-7.2) | 6.5 (4.8-7.7) |
| GT 1a (%) | n/a | 90 | 88 | n/a |
| GT 3 (%) | 100 | n/a | n/a | 76 |
| IL28B CC/CT/TT | 3/6/1 | 2/5/3 | 11/12/2 | 11/12/2 |
| IL28B CC (n, %) | 3 (30) | 2 (20) | 11 (44) | 11 (44) |

A summary of the patient results for treatment-naïve HCV GT2/GT3 Groups 1-5 as related to the percentage of patients having an amount of HCV RNA below the limits of detection (LOD) is provided in **Table 9.**

**Table 9. ELECTRON Groups 1-5 Patient Results**

| Time (Wks) | GS-7977 RBV NO PEG | | GS-7977 RBV 4 wks PEG | | GS-7977 RBV 8 wks PEG^{a} | | GS-7977 RBV 12 weeks PEG^{a} | | GS-7977 NO RBV NO PEG | |
|---|---|---|---|---|---|---|---|---|---|---|
| | (Group 1) | | (Groups 2, 3, 4) | | | | | | (Group 5) | |
| | n/N | %<LOD | n/N | %<LOD | n/N | %<LOD | n/N | %<LOD | n/N | %<LOD |
| 0 | 0/10 | 0 | 0/9 | 0 | 0/10 | 0 | 0/11 | 0 | 10/0 | 0 |
| 4 | 10/10 | 100 | 9/9 | 100 | 10/10 | 100 | 11/11 | 100 | 10/10 | 100 |
| 8 | 10/10 | 100 | 9/9 | 100 | 10/10 | 100 | 11/11 | 100 | 10/10 | 100 |
| 12 | 10/10 | 100 | 9/9 | 100 | 10/10 | 100 | 11/11 | 100 | 10/10 | 100 |
| SVR-4 | 10/10 | 100 | 9/9 | 100 | 10/10 | 100 | 11/11 | 100 | 6/10 | 60 |
| SVR-8 | 10/10 | 100 | 9/9 | 100 | 10/10 | 100 | 11/11 | 100 | 6/10 | 60 |
| SVR-12 | 10/10 | 100 | 9/9 | 100 | 10/10 | 100 | 11/11 | 100 | 6/10 | 60 |
| SVR-24 | 10/10 | 100 | 9/9 | 100 | 10/10 | 100 | 11/11 | 100 | 6/10 | 60 |

From **Table 9** it can be seen that all treatment-naive HCV GT2 and GT3 patients treated with GS-7977 and RBV for 12 weeks (Groups 1-4) had no detectable amount of HCV RNA during the entire treatment period (with or without PEG). All such patients treated with a combination of GS-7977 and RBV (with or without PEG) had no detectable amount of HCV RNA at 12 weeks and at 24 weeks after the termination of treatment.

**Table 9** also reveals that all HCV GT2/GT3 treatment-naive patients receiving 12 weeks of GS-7977 (400 mg QD) monotherapy (Group 5) had no detectable amount of HCV RNA during the entire treatment period. However, only 60% of the patients receiving GS-7977 monotherapy achieved SVR-12 and SVR-24.

Comparing Group 1 (GS-7977 + RBV) with Group 5 (GS-7977 monotherapy), the combination of GS-7977 and ribavirin appears to provide a synergistic increase in SVR-4, SVR-8, SVR-12 and SVR-24 rates, as ribavirin alone has been reported to have little to no effect on HCV RNA levels.

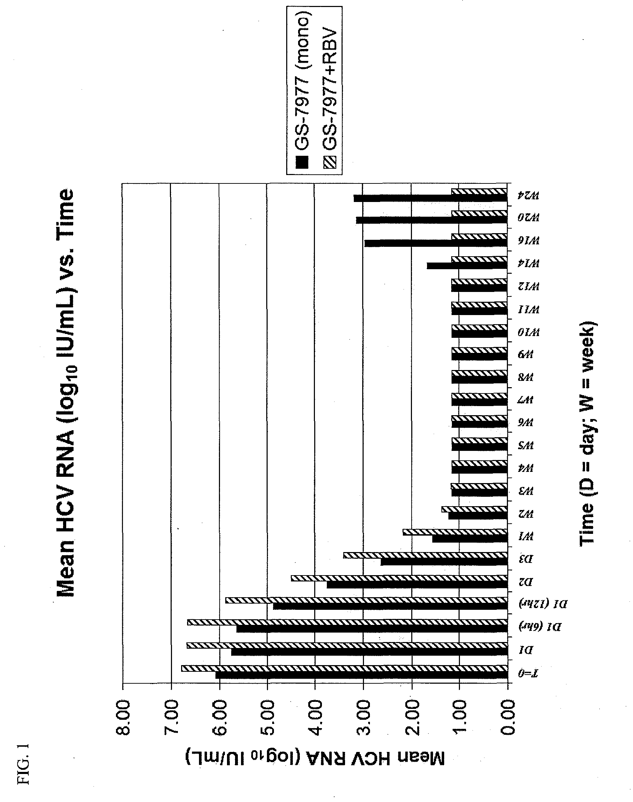

**Table 10** provides the mean HCV RNA values (log₁₀ IU/mL) for treatment-naive HCV GT2/GT3 patients (N=10) for time of treatment (12 weeks) up to 12 weeks after treatment (W24) for patients receiving a combination of 400 mg QD of GS-7977 and 1000/1200 mg BID (based on weight) of RBV (Group 1). **Table 10** also provides the mean HCV RNA values (log₁₀ IU/mL) for treatment-naive HCV GT2/GT3 patients (N=10) for the time of treatment (12 weeks) for patients receiving a 12-week regimen of 400 mg QD of GS-7977 only (Group 5). The terms "D1 (6 hr)" and "D1 (12 hr)" refer to the recorded measurements made 6 hrs and 12 hrs, respectively, on day 1 following day 1 dosing. The data presented in **Table 10** is also illustrated in Figure 1.

**Table 10. ELECTRON Groups 1 and 5 HCV RNA values (log₁₀IU/mL)**

| Time | HCV RNA (log₁₀ IU/mL) | |
|---|---|---|
| | GS-7977 RBV (Group 1) | GS-7977 NO RBV (Group 5) |
| T=0^{a} | 6.79 | 6.08 |
| D1 | 6.67 | 5.74 |
| D1 (6 hr)^{b} | 6.65 | 5.63 |
| D1 (12 hr)^{c} | 5.86 | 4.98 |
| D2 | 4.50 | 3.75 |
| D3 | 3.41 | 2.62 |
| W1 | 2.16 | 1.56 |
| W2 | 1.36 | 1.22 |
| W3 | 1.18 | 1.15 |
| W4 | 1.15 | 1.15 |
| W5 | 1.15 | 1.15 |
| W6 | 1.15 | 1.15 |
| W7 | 1.15 | 1.15 |
| W8 | 1.15 | 1.15 |
| W9 | 1.15 | 1.15 |
| W10 | 1.15 | 1.15 |
| W11 | 1.15 | 1.15 |
| W12 | 1.15 | 1.15 |
| W14 | 1.15 | 1.66 |
| W16 | 1.15 | 2.95 |
| W20 | 1.15 | 3.12 |
| W24 | 1.15 | 3.17 |
| ^{a}Initial Screening Values for patients. | | |
| ^{b}Day 1 results 6 hrs after dosing. | | |
| ^{c}Day 1 results 12 hrs after dosing. | | |

The data in **Table 10** and Figure 1 clearly show that treatment of HCV GT2/GT3 treatment-naive patients with a combination of GS-7977 and RBV (in the amounts noted above) results in mean HCV RNA levels below the limit of detection during weeks 4-12 of the treatment period, as well as SVR-12. This data also shows that the mean HCV RNA value is below the limit of detection during weeks 3-12 of the treatment period for patients receiving GS-7977 monotherapy. However, **Table 10** and Figure 1 also illustrate that patients who received a combination of GS-7977 and ribavirin for 12 weeks (Group 1) maintained lower mean HCV RNA levels for the 12 weeks following cessation of treatment compared to patients who received monotherapy with GS-7977 (Group 5).

These results demonstrate that the combination of GS-7977 and ribavirin is advantageous in that patients can be treated for HCV without receiving peginterferon treatment and achieve a high rate of SVR-12.

A summary of the preliminary patient results for all nine fully reported cohorts of the ELECTRON trial as related to the percentage of patients having an amount of HCV RNA below the limits of detection (LOD) is summarized in **Table 11**.

**Table 11. ELECTRON Groups 1-9 Patient Results**

| Time (Wks) | Genotype 2/3 Treatment Naive | | | | Genotype 1 Null Responders | Genotype 1 | Genotype 2/3 Treatment Experienced |
|---|---|---|---|---|---|---|---|
| | GS-7977 RBV NO PEG 12 wks (Group 1) (N=10) n (%) | GS-7977 RBV PEG 12 wks (Groups 2, 3, 4) (N = 30) n (%) | GS-7977 NO RBV NO PEG 12 weeks (Group 5) (N=10) n (%) | GS-7977 RBV PEG 8 weeks (Group 6) (N=10) n (%) | GS-7977 RBV NO PEG 12 weeks (Group 7) (N = 10) n (%) | GS-7977 RBV NO PEG 12 weeks (Group 8) (N = 25) n (%) | GS-7977 RBV NO PEG 12 weeks (Group 9) (N = 25) n (%) |
| 0 | 0 | 0 | 0 | 0 | 0 | 0 | 0 |
| 1 | 2 (20) | 8 (27) | 5 (50) | 6 (60) | 1 (10) | 8 (32) | 8 (32) |
| 2 | 8 (80) | 23 (77) | 8 (80) | 10 (100) | 7 (70) | 17 (68) | 21 (84) |
| 3 | 9 (90) | 25 (83) | 10 (100) | 10 (100) | 10 (100) | 22 (88) | 25 (100) |
| 4 | 10 (100) | 30 (100) | 10 (100) | 10 (100) | 10 (100) | 25 (100) | 25 (100) |
| 8 | 10 (100) | 30 (100) | 10 (100) | N/A | 10 (100) | 25 (100) | 25 (100) |
| 12 | 10 (100) | 30 (100) | 10 (100) | 10 (100) | 10 (100) | 25 (100) | 25 (100) |
| SVR-4 | 10 (100) | 30 (100) | 6 (60) | 10 (100) | 1 (10) | 22 (88) | 19 (76) |
| SVR-12 | 10 (100) | 30 (100) | 6 (60) | 10 (100) | 1 (10) | 21 (84) | 17 (68) |

The data in **Table 11** demonstrate an SVR-12 rate of 100% for treatment-naive patients with HCV GT2/GT3 (Groups 1-4, 6) when treated with a combination of GS-7977 (400 mg QD) and RBV, regardless of the presence of peginterferon. The data in **Table 11** also demonstrates an SVR-12 rate of 84% for patients with HCV GT1 (Group 8) treated with a combination of GS-7977 and RBV in the absence of peginterferon. In contrast, monotherapy with GS-7977 (Group 5) for GT2/GT3 treatment-naive patients produced an SVR-12 rate of 60%.

All patients enrolled in Group 10 (8 weeks of GS-7977 + ribavirin combination therapy in treatment-naive GT2/GT3 HCV subjects) achieved rapid virological response, and there were no discontinuations or on-treatment breakthroughs.

Treating a subject infected with HCV by administering an effective amount of GS-7977, either alone or in combination with an effective amount of RBV, means that the side-effects normally associated with peginterferon may be avoided. **Table 12** presents adverse events reported in at least 15% of the subjects in any treatment group for ELECTRON Groups 1-9.

**Table 12. ELECTRON Groups 1-9 Adverse Events Reported in at Least 15% of Subjects in Any Treatment Group**

| Adverse Event | GS-7977 RBV NO PEG 12 wks N = 70 (Groups 1, 7, 8, 9) | GS-7977 PEG RBV 12 wks N = 30 (Groups 2, 3, 4) | GS-7977 NO RBV NO PEG 12 wks N = 10 (Group 5) | GS-7977 RBV PEG 8 wks N = 10 (Group 6) |
|---|---|---|---|---|
| ≥1 AE: n (%) | 69 (99) | 30 (100) | 10 (100) | 10 (100) |
| Blood and Lymphatic System Disorders | 10 (14) | 10 (33) | 0 | 3 (30) |
| Anemia | 3 (4) | 5 (17) | 0 | 3 (30) |
| Gastrointestinal Disorders | 32 (46) | 17 (57) | 8 (80) | 7 (70) |
| Nausea | 18 (26) | 9 (30) | 3 (30) | 2 (20) |
| Diarrhoea | 10 (14) | 4 (13) | 0 | 3 (30) |
| Abdominl Pain | 1 (1) | 1 (3) | 0 | 2 (20) |
| Flatulence | 1 (1) | 0 | 0 | 2 (20) |
| General Disorders and Administration Site Conditions | 43 (61) | 22 (73) | 8 (80) | 10 (100) |
| Fatigue | 27 (39) | 11 (37) | 3 (30) | 7 (70) |
| Irritability | 8 (11) | 5 (17) | 1 (10) | 2 (20) |
| Pyrexia | 1 (1) | 4 (13) | 0 | 5 (50) |
| Pain | 1 (1) | 2 (7) | 0 | 2 (20) |
| Chills | 0 | 2 (7) | 0 | 2 (20) |
| Injection Site Erythema | 0 | 1 (3) | 0 | 2 (20) |
| Axillary Pain | 0 | 0 | 2 (20) | 0 |
| Infections and Infestations | 33 (47) | 12 (40) | 5 (50) | 6(60) |
| Upper Respiratory Tract Infection | 11 (16) | 3 (10) | 2 (20) | 1 (10) |
| Metabolism and Nutrition Disorders | 5 (7) | 11 (37) | 0 | 50 (50) |
| Decreased Appetite | 4 (6) | 5 (17) | 0 | 50 (50) |
| Musculoskeletal and Connective Tissue Disorders | 23 (33) | 19 (63) | 2 (20) | 7 (70) |
| Myalgia | 10 (14) | 9 (30) | 1 (10) | 4 (40) |
| Back Pain | 3 (4) | 4 (13) | 1 (10) | 2 (20) |
| Arthralgia | 4 (6) | 5 (17) | 0 | 1 (10) |
| Nervous System Disorders | 40 (57) | 26 (87) | 9 (90) | 7 (70) |
| Headache | 28 (40) | 24 (80) | 8 (80) | 6 (60) |
| Dizziness | 7 (10) | 9 (30) | 2 (20) | 1 (10) |
| Dizziness Postural | 0 | 0 | 0 | 2 (20) |
| Psychiatric Disorders | 26 (37) | 23 (77) | 6(60) | 5 (50) |
| Insomnia | 15 (21) | 16 (53) | 6(60) | 1 (10) |
| Respiratory, Thoracic and Mediastinal Disorders | 18 (26) | 15 (50) | 3 (30) | 5(50) |
| Oropharyngeal Pain | 5 (7) | 3 (10) | 2 (20) | 1 (10) |
| Dyspnoea | 2 (3) | 5 (17) | 0 | 1 (10) |
| Skin and Subcutaneous Tissue Disorders | 31 (44) | 25 (83) | 3 (30) | 8 (80) |
| Rash | 16 (23) | 9 (30) | 1 (10) | 5 (50) |
| Pruritus | 4 (6) | 8 (27) | 0 | 2 (20) |
| Dry Skin | 7 (10) | 5 (17) | 0 | 2 (20) |
| Alopecia | 0 | 5 (17) | 0 | 1 (10) |

The data in **Table 12** reveal that lower incidence rates (%) were reported for a number of types of adverse events for treatment regimens involving the combination of GS-7977 and ribavirin (Groups 1, 7, 8, 9) compared to treatment regimens also involving peginterferon (Groups 2, 3, 4). For example, reduced rates of the following adverse events were reported for the interferon-free treatment regimens combining GS-7977 and ribavirin: blood and lymphatic system disorders (including anemia); pain and chills; metabolism and nutrition disorders (including decreased appetite); musculoskeletal and connective tissue disorders (including myalgia, back pain and arthralgia); nervous system disorders (including headache and dizziness); psychiatric disorders (including insomnia); respiratory, thoracic and mediastinal disorders (including dyspnoea); and skin and subcutaneous tissue disorders (including pruritis, dry skin and alopecia).

The data in **Table 13**, below, reveals reduced frequencies of Grade 3 and Grade 4 hematologic abnormalities for interferon-free Groups 1,5, 7, 8 and 9 compared to Groups 2, 3, 4 and 6 receiving treatment regimens including peginterferon:

**Table 13. ELECTRON Groups 1-9 Reported Grade 3/4 Hematologic Abnormalities**

| Laboratory Abnormalities | GS-7977 RBV NO PEG 12 wks (Group 1) (N=10) n (%) | GS-7977 RBV PEG 12 wks (Groups 2, 3, 4) (N = 30) n (%) | GS-7977 NO RBV NO PEG 12 weeks (Group 5) (N = 10) n (%) | GS-7977 RBV PEG 8 weeks (Group 6) (N=10) n (%) | GS-7977 RBV NO PEG 12 weeks (Group 7) (N = 10) n (%) | GS-7977 RBV NO PEG 12 weeks (Group 8) (N = 25) n (%) | GS-7977 RBV NO PEG 12 weeks (Group 9) (N = 25) n (%) |
|---|---|---|---|---|---|---|---|
| Alanine aminotransferase | | | | | | | |
| Grade 3 | 0 | 1 (3) | 0 | 0 | 0 | 1 (4) | 0 |

| Hemoglobin | | | | | | | |
|---|---|---|---|---|---|---|---|
| Grade 3 | 0 | 1 (3) | 0 | 1 (10) | 1 (10) | 0 | 0 |

| Lymphocytes | | | | | | | |
|---|---|---|---|---|---|---|---|
| Grade 3 | 0 | 3 (10) | 0 | 0 | 0 | 0 | 0 |
| Grade 4 | 0 | 0 | 1 (10) | 0 | 0 | 1 (4) | 0 |

| Neutropenia | | | | | | | |
|---|---|---|---|---|---|---|---|
| Grade 3 | 0 | 5 (17) | 0 | 2 (20) | 0 | 0 | 0 |
| Grade 4 | 0 | 5 (17) | 0 | 0 | 0 | 0 | 0 |

| White blood cells | | | | | | | |
|---|---|---|---|---|---|---|---|
| Grade 3 | 0 | 6 (20) | 0 | 0 | 0 | 0 | 0 |

| INR | | | | | | | |
|---|---|---|---|---|---|---|---|
| Grade 3 | 1 (10) | 0 | 0 | 0 | 1 (10) | 0 | 0 |

Additional results, not shown here, show a rapid normalization of ALT levels in all patients in ELECTRON Groups 1-5 during the treatment period (12 weeks), and to the extent of available data, for periods after the end of the treatment period.

### GS-7977 Resistance in Human Clinical Studies

To date, no virologic breakthrough has been observed during treatment with GS-7977, suggesting a high barrier to resistance. Across the P7977-0221, PROTON, ELECTRON (Groups 1-9) and ATOMIC Phase 2 human clinical studies of treatment regimens involving GS-7977 alone or in combination with ribavirin and/or peginterferon, 53 out of 621 patients have experienced viral relapse after cessation of GS-7977-containing treatment. Population sequencing of the viral relapse samples showed that S282T was detected in only one of the 53 patients, who was GT2b and relapsed 4 weeks after completion of 12 weeks of GS-7977 monotherapy. Deep sequencing revealed 99% S282T in this GT2b patient at relapse. Population and clonal phenotypic analysis demonstrated that the GT2b S282T-containing sample was 8- to 13-fold less susceptible to GS-7977 compared to corresponding baseline virus. For the other 52 patients experiencing relapse, deep sequencing at baseline and relapse showed no S282T, and no specific NS5B mutation at other residues was identified by population or deep sequencing as being associated with GS-7977 resistance. (*See also* Svarovskaia et al., 63rd Annual Meeting of the American Association for the Study of Liver Diseases, Poster 753, Nov. 11, 2012.)

The foregoing illustrates that GS-7977 has a high resistance barrier. Notably, the S282T mutation has not been observed in any patient receiving a treatment regimen combining GS-7977 and ribavirin.

### Concordance of SVR-4 with SVR-12 and SVR -24 for Treatment Regimens Combining GS-7977 with Ribavirin and Optionally Peginterferon

Florian et al. have reported that SVR-12 and SVR-24 were concordant across a large population database of HCV clinical trials including trials involving peginterferon/ribavirin combination treatment and treatment regimens combining peginterferon, ribavirin and telaprevir or boceprevir, with SVR-12 having a positive predictive value of 98% for SVR-24. (Florian et al., AASLD 2011, Abstract LB-28; *see also* Martinot-Peignoux et al., Hepatology (2010) 51(4): 1122-1126.)

HCV data from treatment-naive GT1, GT2 and GT3 patients in the PROTON, ELECTRON and ATOMIC Phase 2 studies who received at least 12 weeks of treatment with GS-7977, either alone or in combination with ribavirin and optionally peginterferon, were evaluated. Only patients treated for at least 12 weeks with 400 mg GS-7977 who had SVR-4 and SVR-12 or SVR-4 and SVR-24 data were included in the analysis (259 of 596 patients). The analysis found 99-100% concordance between SVR-4 and both SVR-12 and SVR-24 across all regimens for patients who achieved SVR-4 and for whom posttreatment week 12 data were available. These results show that SVR-4 is highly concordant with SVR-12 and SVR-24 for GT1, GT3 and GT3 HCV patients treated with 400 mg GS-7977 and ribavirin, and optionally with peginterferon. (Lawitz et al., GS-7977 Phase 2 Trials: Concordance of SVR4 with SVR12 and SVR24 in HCV Genotypes 1-3, EASL (April 18-22, 2012).)

The foregoing suggests that the SVR data presented herein may have predictive value for longer-term SVR rates including SVR-24, SVR-36 and SVR-48.

The compositions and unit dosage forms comprising GS-7977 disclosed herein provide good stability to moisture and degradation, as well as desirable dissolution and disintegration profiles. They may be used to treat HCV infection optionally in combination with ribavirin, peginterferon or any other antiviral agent.

Additionally, the foregoing data illustrate that GS-7977 administered in combination with ribavirin (with or without peginterferon) elicited a rapid decline in HCV RNA and end of treatment response (EOTR) in patients with HCV GT1, GT2 and GT3. No viral breakthrough has been observed during the course of treatment with GS-7977, including when combined with ribavirin and optionally peginterferon. SVR-12 was 100% for HCV GT2 and GT3 treatment-naive patients who received a combination of GS-7977 and ribavirin for 12 weeks and 84% for HCV GT1 treatment-naive patients who received a combination of GS-7977 and ribavirin for 12 weeks, compared to 60% SVR-12 for HCV GT2 and GT3 treatment-naive patients who received GS-7977 alone. Given that ribavirin, alone, has been shown to have little to no effect on HCV RNA levels in human clinical trials, the foregoing clinical and *in vitro* data demonstrates that the combination of GS-7977 and ribavirin produces a synergistic reduction in HCV RNA levels.

Further, treatment arms in the ELECTRON trial receiving GS-7977 in combination with ribavirin, compared to treatment arms also receiving peginterferon, reported reduced incidences of side effects, suggesting that interferon-free treatment with a combination of GS-7977 and ribavirin may offer advantages over treatment regimens involving peginterferon.

Even further, *in vitro* results showing that HCV replicons with the S282T mutation, which show reduced susceptibility to GS-7977, display increased susceptibility to ribavirin suggest that the combination of GS-7977 and ribavirin may provide a treatment regimen resulting in reduced rates of resistance compared to monotherapy with GS-7977. Thus far, the S282T mutation has not been observed in a patient receiving GS-7977 and ribavirin combination therapy, compared to the observation of the mutation in one patient receiving GS-7977 monotherapy.

The ability to provide effective therapy without peginterferon according to the methods described herein has the potential to significantly improve therapeutic options for individuals living with HCV infection.

The foregoing description of the present invention provides illustration and description, but is not intended to be exhaustive or to limit the invention to the precise one disclosed. Modifications and variations are possible in light of the above teachings or may be acquired from practice of the invention.

## Claims

1. A pharmaceutical composition comprising:
a) about 25% to about 35% w/w of crystalline GS-7977 having the structure 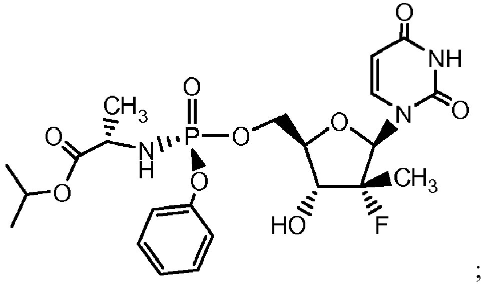 and
b) a pharmaceutically acceptable excipient comprising:
i) about 30% w/w of mannitol and about 30% w/w of microcrystalline cellulose;
ii) about 5 %w/w of croscarmellose sodium;
iii) about 0.5% w/w of colloidal silicon dioxide; and
iv) about 1.5% w/w of magnesium stearate,
wherein the crystalline GS-7977 has XRPD 2θ-reflections (°) at about: 6.1, 8.2, 10.4, 12.7, 17.2, 17.7, 18.0, 18.8, 19.4, 19.8, 20.1, 20.8, 21.8, and 23.3.

2. A unit dosage form comprising:
a) about 400 mg of crystalline GS-7977 having the structure 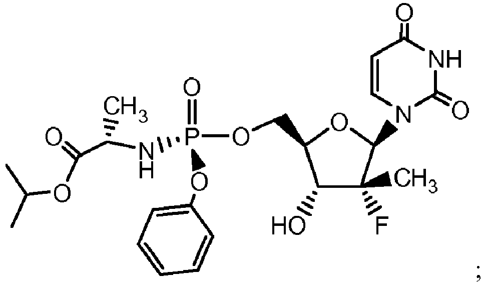 and
b) a pharmaceutically acceptable excipient comprising:
i) about 660 mg to about 780 mg of a diluent;
ii) about 30 mg to about 90 mg of a disintegrant;
iii) about 3 mg to about 9 mg of a glidant; and
iv) about 15 mg to about 21 mg of a lubricant,
wherein the crystalline GS-7977 has XRPD 20-reflections (°) at about: 6.1, 8.2, 10.4, 12.7, 17.2, 17.7, 18.0, 18.8, 19.4, 19.8, 20.1, 20.8, 21.8, and 23.3.

3. A unit dosage form according to claim 2, wherein the diluent is selected from the group consisting of dicalcium phosphate, cellulose, compressible sugars, dibasic calcium phosphate dehydrate, lactose, mannitol, microcrystalline cellulose, starch, tribasic calcium phosphate, and combinations thereof.

4. A unit dosage form according to claim 3, wherein the diluent is selected from the group consisting of mannitol, microcrystalline cellulose, and combinations thereof.

5. A unit dosage form according to claim 2, wherein the disintegrant is selected from the group consisting of croscarmellose sodium, crospovidone, microcrystalline cellulose, modified corn starch, povidone, pregelatinized starch, sodium starch glycolate, and combinations thereof.

6. A unit dosage form according to claim 5, wherein the disintegrant is croscarmellose sodium.

7. A unit dosage form according to claim 2, wherein the glidant is selected from the group consisting of colloidal silicon dioxide, talc, starch, starch derivatives, and combinations thereof.

8. A unit dosage form according to claim 7, wherein the glidant is colloidal silicon dioxide.

9. A unit dosage form according to claim 2, wherein the lubricant is selected from the group consisting of calcium stearate, magnesium stearate, polyethylene glycol, sodium stearyl fumarate, stearic acid, talc, and combinations thereof.

10. A unit dosage form according to claim 9, wherein the lubricant is magnesium stearate.

11. A pharmaceutical composition according to claim 1 or a unit dosage form according to claim 2 further comprising a coating agent.

12. A unit dosage form according to claim 2, wherein the unit dosage form comprises:
a) about 400 mg of crystalline GS-7977;
b) about 360 mg of mannitol and about 356 mg of microcrystalline cellulose;
c) about 60 mg of croscarmellose sodium;
d) about 6 mg of colloidal silicon dioxide; and
e) about 18 mg of magnesium stearate.

13. A unit dosage form according to claim 2, wherein the unit dosage form comprises a capsule or a tablet.

14. A pharmaceutical composition according to claim 1 or a unit dosage form according to claim 2 for use in the treatment of hepatitis C virus infection in a human.

15. A pharmaceutical composition for use or a unit dosage form for use according to claim 14 for use in combination with ribavirin.

16. A pharmaceutical composition for use or a unit dosage form for use according to claim 15, wherein the use in combination with ribavirin is as part of an interferon-free treatment regimen.

17. A pharmaceutical composition for use or a unit dosage form for use according to claim 14, wherein the hepatitis C virus is HCV genotype 1, 2, 3, 4 or any combination thereof.

18. A pharmaceutical composition for use or a unit dosage form for use according to claim 17 wherein the hepatitis C virus is HCV genotype 4.

## Patentansprüche

1. Pharmazeutische Zusammensetzung, umfassend:
a) etwa 25% bis etwa 35% Gew./Gew. kristallines GS-7977, das die Struktur aufweist: 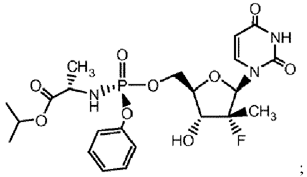 und
b) einen pharmazeutisch verträglichen Hilfsstoff, umfassend:
i) etwa 30% Gew./Gew. Mannit und etwa 30% Gew./Gew. mikrokristalline Cellulose;
ii) etwa 5% Gew./Gew. Croscarmellose-Natrium;
iii) etwa 0,5% Gew./Gew. kolloidales Siliciumdioxid; und
iv) etwa 1,5% Gew./Gew. Magnesiumstearat,
wobei das kristalline GS-7977 XRPD 2θ-Reflexionen (°) bei etwa 6,1, 8,2, 10,4, 12,7, 17,2, 17,7, 18,0, 18,8, 19,4, 19,8, 20,1, 20,8, 21,8 und 23,3 aufweist.

2. Einheitsdosierungsform, umfassend:
a) etwa 400 mg kristallines GS-7977, das die Struktur aufweist: 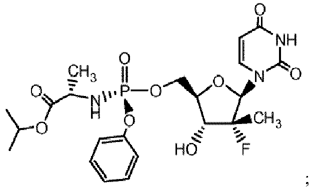 und
b) einen pharmazeutisch verträglichen Hilfsstoff, umfassend:
i) etwa 660 mg bis etwa 780 mg eines Streckmittels;
ii) etwa 30 mg bis etwa 90 mg eines Zerfallsmittels;
iii) etwa 3 mg bis etwa 9 mg eines Gleitmittels; und
iv) etwa 15 mg bis etwa 21 mg eines Schmiermittels;
wobei das kristalline GS-7977 XRPD 2θ-Reflexionen (°) bei etwa 6,1, 8,2, 10,4, 12,7, 17,2, 17,7, 18,0, 18,8, 19,4, 19,8, 20,1, 20,8, 21,8 und 23,3 aufweist.

3. Einheitsdosierungsform nach Anspruch 2, wobei das Streckmittel ausgewählt ist aus der Gruppe bestehend aus Dicalciumphosphat, Cellulose, komprimierbaren Zuckern, zweibasischem Calciumphosphat-Dihydrat, Lactose, Mannit, mikrokristalliner Cellulose, Stärke, dreibasischem Calciumphosphat, und Kombinationen davon.

4. Einheitsdosierungsform nach Anspruch 3, wobei das Streckmittel ausgewählt ist aus der Gruppe bestehend aus Mannit, mikrokristalliner Cellulose und Kombinationen davon.

5. Einheitsdosierungsform nach Anspruch 2, wobei das Zerfallsmittel ausgewählt ist aus der Gruppe bestehend aus Croscarmellose-Natrium, Crospovidon, mikrokristalliner Cellulose, modifizierter Maisstärke, Povidon, vorgelatinierter Stärke, Natriumstärkeglykolat und Kombinationen davon.

6. Einheitsdosierungsform nach Anspruch 5, wobei das Zerfallsmittel ausgewählt ist aus der Gruppe bestehend aus Croscarmellose-Natrium.

7. Einheitsdosierungsform nach Anspruch 2, wobei das Gleitmittel ausgewählt ist aus der Gruppe bestehend aus kolloidalem Siliciumdioxid, Talk, Stärke, Stärkederivaten und Kombinationen davon.

8. Einheitsdosierungsform nach Anspruch 7, wobei das Gleitmittel kolloidales Siliciumdioxid ist.

9. Einheitsdosierungsform nach Anspruch 2, wobei das Schmiermittel ausgewählt ist aus der Gruppe bestehend aus Calciumstearat, Magnesiumstearat, Polyethylenglykol, Natriumstearylfumarat, Stearinsäure, Talk und Kombinationen davon.

10. Einheitsdosierungsform nach Anspruch 9, wobei das Schmiermittel Magnesiumstearat ist.

11. Pharmazeutische Zusammensetzung nach Anspruch 1 oder eine Einheitsdosierungsform nach Anspruch 2, ferner umfassend ein Beschichtungsmittel.

12. Einheitsdosierungsform nach Anspruch 2, wobei die Einheitsdosierungsform umfasst:
a) etwa 400 mg kristallines GS-7977;
b) etwa 360 mg Mannit und etwa 356 mg mikrokristalline Cellulose;
c) etwa 60 mg Croscarmellose-Natrium;
d) etwa 6 mg kolloidales Siliciumdioxid; und
e) etwa 18 mg Magnesiumstearat.

13. Einheitsdosierungsform nach Anspruch 2, wobei die Einheitsdosierungsform eine Kapsel oder eine Tablette umfasst.

14. Pharmazeutische Zusammensetzung nach Anspruch 1 oder eine Einheitsdosierungsform nach Anspruch 2 zur Verwendung in der Behandlung von Hepatitis C-Virusinfektion in einem Menschen.

15. Pharmazeutische Zusammensetzung zur Verwendung oder eine Einheitsdosierungsform zur Verwendung nach Anspruch 14 zur Verwendung in Kombination mit Ribavirin.

16. Pharmazeutische Zusammensetzung zur Verwendung oder eine Einheitsdosierungsform zur Verwendung nach Anspruch 15, wobei die Verwendung in Kombination mit Ribavirin ein Teil eines interferonfreien Behandlungsschemas ist.

17. Pharmazeutische Zusammensetzung zur Verwendung oder eine Einheitsdosierungsform zur Verwendung nach Anspruch 14, wobei das Hepatitis C-Virus vom HCV-Genotyp 1, 2, 3, 4 oder eine beliebige Kombination davon ist.

18. Pharmazeutische Zusammensetzung zur Verwendung oder eine Einheitsdosierungsform zur Verwendung nach Anspruch 17, wobei das Hepatitis C-Virus vom HCV-Genotyp 4 ist.

## Revendications

1. Composition pharmaceutique comprenant :
a) environ 25 % à environ 35 % poids/poids de GS-7977 cristallin présentant la structure : 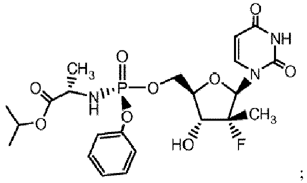 et
b) un excipient pharmaceutiquement acceptable comprenant :
i) environ 30 % poids/poids de mannitol et environ 30 % poids/poids de cellulose microcristalline ;
ii) environ 5 % poids/poids de croscarmellose de sodium ;
iii) environ 0,5 % poids/poids de dioxyde de silicium colloïdal ; et
v) environ 1,5 % poids/poids de stéarate de magnésium,
dans laquelle le GS-7977 cristallin présente des réflexions 2θ XRPD (°) à environ :
6,1, 8,2, 10,4, 12,7, 17,2, 17,7, 18,0, 18,8, 19,4, 19,8, 20,1, 20,8, 21,8 et 23,3.

2. Forme de dosage unitaire comprenant :
a) environ 400 mg de GS-7977 cristallin présentant la structure : 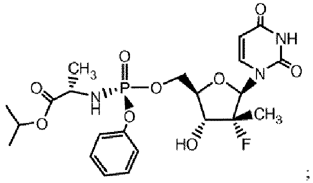 et
b) un excipient pharmaceutiquement acceptable comprenant :
i) environ 660 mg à environ 780 mg d'un diluant ;
ii) environ 30 mg à environ 90 mg d'un agent de désagrégation ;
iii) environ 3 mg à environ 9 mg d'un agent de glissement ; et
iv) environ 15 mg à environ 21 mg d'un agent de lubrification,
dans laquelle le GS-7977 cristallin présente des réflexions 2θ XRPD (°) à environ :
6,1, 8,2, 10,4, 12,7, 17,2, 17,7, 18,0, 18,8, 19,4, 19,8, 20,1, 20,8, 21,8 et 23,3.

3. Forme de dosage unitaire selon la revendication 2, dans laquelle le diluant est sélectionné parmi le groupe qui est constitué par le phosphate de dicalcium, la cellulose, les sucres compressibles, le phosphate de calcium dibasique dihydraté, le lactose, le mannitol, la cellulose microcristalline, l'amidon, le phosphate de calcium tribasique et des combinaisons de ceux-ci.

4. Forme de dosage unitaire selon la revendication 3, dans laquelle le diluant est sélectionné parmi le groupe qui est constitué par le mannitol, la cellulose microcristalline et des combinaisons de ceux-ci.

5. Forme de dosage unitaire selon la revendication 2, dans laquelle l'agent de désagrégation est sélectionné parmi le groupe qui est constitué par la croscarmellose de sodium, la crospovidone, la cellulose microcristalline, l'amidon de maïs modifié, la povidone, l'amidon prégélatinisé, le glycolate d'amidon de sodium et des combinaisons de ceux-ci.

6. Forme de dosage unitaire selon la revendication 5, dans laquelle l'agent de désagrégation est la croscarmellose de sodium.

7. Forme de dosage unitaire selon la revendication 2, dans laquelle l'agent de glissement est sélectionné parmi le groupe qui est constitué par le dioxyde de silicium colloïdal, le talc, l'amidon, les dérivés d'amidon et des combinaisons de ceux-ci.

8. Forme de dosage unitaire selon la revendication 7, dans laquelle l'agent de glissement est le dioxyde de silicium colloïdal.

9. Forme de dosage unitaire selon la revendication 2, dans laquelle l'agent de lubrification est sélectionné parmi le groupe qui est constitué par le stéarate de calcium, le stéarate de magnésium, le polyéthylène glycol, le stéarylfumarate de sodium, l'acide stéarique, le talc et des combinaisons de ceux-ci.

10. Forme de dosage unitaire selon la revendication 9, dans laquelle l'agent de lubrification est le stéarate de magnésium.

11. Composition pharmaceutique selon la revendication 1 ou forme de dosage unitaire selon la revendication 2, comprenant en outre un agent d'enrobage.

12. Forme de dosage unitaire selon la revendication 2, dans laquelle la forme de dosage unitaire comprend :
a) environ 400 mg de GS-7977 cristallin ;
b) environ 360 mg de mannitol et environ 356 mg de cellulose microcristalline ;
c) environ 60 mg de croscarmellose de sodium ;
d) environ 6 mg de dioxyde de silicium colloïdal ; et
e) environ 18 mg de stéarate de magnésium.

13. Forme de dosage unitaire selon la revendication 2, dans laquelle la forme de dosage unitaire comprend une gélule ou un comprimé.

14. Composition pharmaceutique selon la revendication 1 ou forme de dosage unitaire selon la revendication 2, pour une utilisation dans le traitement d'une infection par le virus de l'hépatite C chez un être humain.

15. Composition pharmaceutique pour une utilisation ou forme de dosage unitaire pour une utilisation selon la revendication 14 pour une utilisation en combinaison avec la ribavirine.

16. Composition pharmaceutique pour une utilisation ou forme de dosage unitaire pour une utilisation selon la revendication 15, dans laquelle l'utilisation en combinaison avec la ribavirine constitue une partie d'un protocole thérapeutique sans interféron.

17. Composition pharmaceutique pour une utilisation ou forme de dosage unitaire pour une utilisation selon la revendication 14, dans laquelle le virus de l'hépatite C est le génotype 1, 2, 3, 4 du HCV ou une quelconque combinaison afférente.

18. Composition pharmaceutique pour une utilisation ou forme de dosage unitaire pour une utilisation selon la revendication 17, dans laquelle le virus de l'hépatite C est le génotype 4 du HCV.
